# EUROPEAN PATENT SPECIFICATION

(11) **EP 1 854 885 B1**
(45) Date of publication and mention of the grant of the patent: **08.09.2010**
(21) Application number: 07101045.8
(22) Date of filing: 20.11.1998
(51) Int. Cl.: C12N 15/31, C12N 15/62, C07K 14/295, C07K 16/12, C07K 19/00, A01K 67/027, A61K 39/118, G01N 33/53, C12Q 1/68

(54) **Chlamydia pneumoniae outer membrane polypeptide, fragments thereof and uses thereof, in particular for the diagnosis, prevention and treatment of infection**
Äusseres Membranpolypeptid von Chlamydia pneumoniae sowie Fragmente davon und deren Verwendung, insbesondere zur Diagnose, Prävention und Behandlung einer Infektion
Polypeptide de la membrane externe de Chlamydia pneumoniae, fragments et leurs utilisations, en particulier pour le diagnostic, la prévention et le traitement de l'infection

(30) Priority: 21.11.1997 FR 9714673; 04.11.1998 US 107078 P
(43) Date of publication of application: 14.11.2007
(62) Divisional of application: 98954662.7
(73) Proprietor: Merck Serono Biodevelopment, 69008 Lyon (FR)
(72) Inventor: Griffais, Rémy, 92120 Montrouge (FR); Hoiseth, Susan K., Suffern, NY 10901 (US); Zagursky, Robert John, Victor, NY 14564 (US); Metcalf, Benjamin J., Rochester, NY 14618 (US); Peek, Joel A., Pittsford, NY 14534 (US); Sankaran, Banumathi, Penfield, NY 14526 (US); Fletcher, Leah Diane, Geneseo, NY 14454 (US)
(74) Representative: Ahner, Francis

(56) References cited:
- PEREZ MELGOSA M ET AL: "Isolation and characterization of a gene encoding a Chlamydia pneumoniae 76-kilodalton protein containing a species-specific epitope." INFECT IMMUN, MAR 1994, 62 (3) P880-6, 1 March 1994 (1994-03-01), XP002076845 UNITED STATES
- TOMB J -F ET AL: "THE COMPLETE GENOME SEQUENCE OF THE GASTRIC PATHOGEN HELICOBACTER PYLORI" NATURE, vol. 388, no. 6642, 7 August 1997 (1997-08-07), pages 539-547, TABEL, XP002062106
- DATABASE Geneseq [Online] 21 May 1997 (1997-05-21), "Chlamydia pneumoniae antigen used as DHFR-linked fusion protein." XP002486559 retrieved from EBI accession no. GSP:AAW10975 Database accession no. AAW10975
- DATABASE UniProt [Online] 1 July 1993 (1993-07-01), "60 kDa chaperonin (Protein Cpn60) (groEL protein)." XP002486560 retrieved from EBI accession no. UNIPROT:P31681 Database accession no. P31681
- KORNAK JM ET AL: "Sequence analysis of the gene encoding the Chlamydia pneumoniae DnaK protein homolog." INFECT IMMUN, FEB 1991, 59 (2) P721-5, 1 February 1991 (1991-02-01), XP002076846 UNITED STATES
- WATSON MW ET AL: "The CrP operon of Chlamydia psittaci and Chlamydia pneumoniae." MICROBIOLOGY, OCT 1995, 141 ( PT 10) P2489-97, 1 October 1995 (1995-10-01), XP002076847 ENGLAND
- LOBAU S ET AL: "Molecular cloning, sequence analysis, and functional characterization of the lipopolysaccharide biosynthetic gene kdtA encoding 3-deoxy-alpha-D-manno-octulosonic acid transferase of Chlamydia pneumoniae strain TW-183." MOL MICROBIOL, NOV 1995, 18 (3) P391-9, 1 November 1995 (1995-11-01), XP002076848 ENGLAND
- PETERSON EM ET AL: "Characterization of the murine antibody response to peptides representing the variable domains of the major outer membrane protein of Chlamydia pneumoniae." INFECT IMMUN, AUG 1996, 64 (8) P3354-9, 1 August 1996 (1996-08-01), XP002076849 UNITED STATES
- DATABASE Geneseq [Online] 13 September 1999 (1999-09-13), "Chlamydia pneumoniae transmembrane protein sequence." XP002486561 retrieved from EBI accession no. GSP:AAY35281 Database accession no. AAY35281
- DATABASE UniProt [Online] 1 November 1996 (1996-11-01), "Peptidyl-prolyl cis-trans isomerase (EC <A HREF="http://srs.ebi.ac.uk/srsbin/cgi-bin/ wgetz?[enzyme-ECNumber:5.2.1.8]+-e">5.2.1. 8</A>)." XP002486562 retrieved from EBI accession no. UNIPROT:Q46176 Database accession no. Q46176
- DATABASE UniProt [Online] 1 October 1994 (1994-10-01), "Outer membrane protein B precursor." XP002486563 retrieved from EBI accession no. UNIPROT:P38006 Database accession no. P38006
- DATABASE Geneseq [Online] 13 September 1999 (1999-09-13), "Chlamydia pneumoniae surface exposed polypeptide." XP002486564 retrieved from EBI accession no. GSP:AAY35504 Database accession no. AAY35504
- DATABASE UniProt [Online] 1 May 1999 (1999-05-01), "Conserved outer membrane lipoprotein protein (Gene encoding a 29 kDa immunogenic protein) (Lipoprotein, putative)." XP002486570 retrieved from EBI accession no. UNIPROT:Q9Z8R0 Database accession no. Q9Z8R0
- DATABASE UniProt [Online] 1 November 1995 (1995-11-01), "Outer membrane lipoprotein 3 precursor (PLP3)." XP002486571 retrieved from EBI accession no. UNIPROT:Q08870 Database accession no. Q08870

## Description

The subject of the invention is the identification genomic sequence from the nucleotide sequence encoding an outer membrane polypeptide of *Chlamydia pneumoniae*, as well as vectors including the said sequence and cells or animals transformed with these vectors. The invention also relates to methods of detecting these nucleic acids or polypeptides and kits for diagnosing *Chlamydia pneumoniae* infection. The invention finally comprises, pharmaceutical, in particular vaccine, compositions for the prevention and/or treatment of *Chlamydia pneumoniae* infections.

Comparative analysis of the sequence of the gene encoding the ribosomal 16S RNA has been widely used for the phylogenetic study of prokaryotes. This approach has made it possible to classify the Chlamydiae among the eubacteria, among which they represent a well-isolated group, with, nevertheless, a very weak link with the planctomyces. The Chlamydiae thus exhibit some unique characteristics within the eubacteria, in particular their development cycle and the structure of their membranes. They have a unique two-phase cell cycle: the elementary body, a small extracellular form, attaches to the host and is phagocytosed; in the phagosome, it is converted to the replicative intracellular form, the reticulate body. The Chlamydiae are obligate intracellular bacteria which multiply in eukaryotic cells at the expense of their energy reserves and nucleotide pools; they are responsible for a wide variety of diseases in mammals and birds. The Chlamydiae are the only members of the order Chlamydiales, of the family Chlamydiaceae and of the genus Chlamydia. Within the genus *Chlamydia,* four species are currently described: *Chlamydia trachomatis, Chlamydia psittaci, Chlamydia pneumoniae* and *Chlamydia pecorum.* These bacteria are grouped together and share biological and biochemical properties. Among them, only the first three infect humans, *Chlamydia pecorum* being a pathogen of ruminants.

The species *Chlamydia psittaci* infects many animals, in particular birds, and is transmissible to humans. It is responsible for atypical pneumonia, for hepatic and renal dysfunction, for endocarditis and for conjunctivitis.

The species *Chlamydia trachomatis* is the best characterized. Besides a murine strain, it is divided into two groups which are distinguishable by the nature of the diseases for which they are responsible: trachoma, genital attack and venereal lymphogranulomatosis. There are fifteen human serotypes of *Chlamydia trachomatis* (A, K) and LGV (L1, L2, L3). Strains A to C are mainly found in eye infections, whereas strains D to K and LGV are essentially responsible for genital entry infections. It should be mentioned that the LGV strains are responsible for systemic diseases. Historically, it was in 1906 that Halberstaeder and Von Provaseck discovered, in trachoma patients, the presence of inclusions in the cytoplasm of the cells derived from conjunctival scrapings. In 1940, Rake and Jones described these same inclusions in certain cells obtained by puncturing the ganglia from a patient suffering from venereal granulomatosis. Characterization of the *Chlamydia trachomatis* microorganism was only successfully carried out in 1957, after a series of isolations in cell cultures.

It was in 1983 that *Chlamydia pneumoniae* was recognized as a human pathogen (Grayston JT et al., 1986); since then, special attention has been paid to this bacterium and it is estimated (Gaydos CA et al., 1994) that 10% of pneumonias, and 5% of bronchitides and sinusites are attributable to *Chlamydia pneumoniae* (Aldous MB et al., 1992). More recently, the association of this bacterium with the pathogenesis of asthmatic disease and of cardiovascular impairments is increasingly of interest.

Serological studies have made it possible to observe that *Chlamydia pneumoniae* infection is common in children between 5 and 16 years of age. Before this age, it is rare to find antibodies; the increase in the number of individuals carrying antibodies is then correlated with age up to 20 years. Accordingly, 50% of adults are carriers of antibodies, it being possible for this prevalence to be as high as 75%. These figures are all the more striking since a first infection induces antibody levels of which the persistence over time is limited to 3 or at most 5 years, which suggests frequent reinfection during the entire lifespan. The annual seroconversion rate is about 8% between 8 and 12 years and about 6% between 12 and 16 years (Haidl et al., 1994). Before the age of 15 years, the seroprevalence of the disease is identical between both sexes. After this age, men are more frequently infected than women; this is true in all regions worldwide where such studies have been carried out.

These infections are geographically highly widespread, as shown by numerous studies carried out throughout the world (Kanamoto Y et al., 1991; Tong CY et al., 1993). Developed countries of the north such as Canada, Denmark and Norway have the lowest infection rates; conversely, the highest prevalence rates are found in the less developed countries of tropical regions where the infection may occur before the age of 5 years.

Humans are the only known reservoir for *Chlamydia pneumoniae* and it is probable that the infection is caused by direct transmission, respiratory secretions probably being responsible for this low-yield transmission (Aldous et al., 1992). The chain of transmission may also appear to be indirect (Kleemola M et al., 1988), suggesting that the infection is caused by an effective transmission, but also that asymptomatic carriers exist, which could explain the high prevalence of the disease. Other studies (Mordhorst CH et al., 1992) show that the efficiency of the transmission varies according to the individuals and list cases of infection affecting all or the majority of members of one family or of a group of families. The period of incubation is several weeks, significantly longer in this regard than that of many other respiratory pathogenic agents. Although under conditions of high relative humidity the infectivity of *Chlamydia pneumoniae* in the open air decreases rapidly, suggesting a direct mode of transmission under these conditions, it is probable that the transmission occurs in some cases indirectly since the microorganism can survive for up to 30 hours in a hostile environment (Falsey et al., 1993).

Clinical manifestations due to *Chlamydia pneumoniae* are essentially respiratory diseases. Pneumonia and bronchitis are the most frequent because they are clinically patent: since etiological diagnosis is evoked in this case, the infectious agent is identified. The asymptomatic diseases are probably numerous (Grayston JT et al., 1992; Grayston JT et al., 1986; Thom DH et al., 1990). The disease then progresses via bronchitis or pneumonia; fever is absent at the time of examination but is sometimes reported by the patient. The degree of seriousness of the disease is variable and in hospitalized patients, it is common to observe pleural effusion; a generalized infection may also be observed and, in severe cases, anatomicopathological examination shows *Chlamydia pneumoniae* diseases.

Other syndromes such as sinusitis (Hashiguchi K et al., 1992), purulent otitis media (Ogawa H et al., 1992), or pharyngitis (Huovinen P et al., 1989) have been described, as well as infections with respiratory impairments similar to asthma (Hahn DL et al., 1991). *Chlamydia pneumoniae* has also been associated with sarcoidosis, with erythema nodosum (Sundelof et al., 1993) and one case of Guillain-Barré syndrome has even been described (Haidl et al., 1992). The involvement of *Chlamydia pneumoniae* in Reiter's syndrome has also been evaluated (Braun J et al., 1994).

The association of *Chlamydia pneumoniae* with coronary diseases and with myocardial infarction was first suspected from the observation of the high antibody level in 71% of patients having a heart disease (Shor A et al., 1992; Kuo CC et al., 1993; Puolakkainen M et al., 1993; Thomas GN et al., 1997). Studies carried out in several countries have shown similar results in patients with atheromatous impairments (Shor A et al., 1992; Kuo CC et al., 1993; Puolakkainen M et al., 1993; Grayston JT et al., 1996; Casas-Ciria J et al., 1996; Thomas GN et al., 1997; Jackson LA et al., 1997) and in patients with carotid impairments. Anatomicopathological and microbiological studies have detected *Chlamydia pneumoniae* in the vessels. The electron microscope has made it possible to visualize the bacterium (Ladany S et al., 1989), which has in fact been demonstrated by other techniques such as PCR (Campbell LA et al., 1992; Kuo CC et al., 1993; Kuo CC et al., 1988). It also appears that the bacterium is more frequently found in old atheromatous lesions. Other studies carried out on young subjects from 15 to 35 years have given the opportunity to study the coronary arteries of people without atherosclerosis, this observation not being possible in older subjects (the onset of the atheromatous disease is early). In these young subjects, the PCR studies did not find *Chlamydia pneumoniae* in subjects free of atheromatous disease, but revealed the presence of *Chlamydia pneumoniae* in two of the eleven subjects who showed early lesions and in six of the seven subjects who developed atheroma plaques. These studies therefore show that the atheroma plaque is very strongly correlated with the presence of *Chlamydia pneumoniae,* but the role played by the bacterium in vascular pathology is not yet defined.

The data relating to controlled clinical studies analysing the effect of treatments in *Chlamydia pneumoniae* infections are limited in number. Unlike penicillin, ampicillin or the sulphamides, erythromycin, tetracycline or doxycycline show an antibiotic activity *in vitro* against *Chlamydia pneumoniae.* However, a treatment at high doses should be continued for several weeks in order to avoid a recurrence of the infection. Accordingly, the use of two new macrolides, clarithromycin and azithromycin, whose diffusion, bioavailability and half-life allow shorter and better tolerated cures, is nowadays preferred. In the absence of definitive proof based on the results of clinical studies, an effective, without recurrences, and well-tolerated treatment of *Chlamydia pneumoniae* infections therefore remains desirable.

An even more important need up until now relates to a specific and sensitive diagnosis, which can be carried out conveniently and rapidly, allowing early screening for the infection. Methods based on *Chlamydia pneumoniae* culture are slow and require a considerable know-how because of the difficulty involved in the collection, preservation and storage of the strain under appropriate conditions. Methods based on antigen detection (EIA, DFA) or on nucleic acid amplification (PCR) provide tests which are more suitable for laboratory practice. A reliable, sensitive and convenient test, which allows distinction between serogroups and a fortiori between *Chlamydia pneumoniae* species is therefore highly desirable.

This is all the more important since the symptoms of *Chlamydia pneumoniae* infection appear slowly, since all the pathologies associated with these infections have not yet been identified, and since, as has been mentioned above, an association is suspected between these infections and serious chronic infections, asthma or atherosclerosis.

No vaccine is yet available against *Chlamydia pneumoniae:* this is due to the labile nature of the antigens specific to the strain, which has so far prevented their specific identification.

Although the number of studies and of animal models developed is high, the antigens used have not induced sufficient projective immunity to lead to the development of human vaccines. In the case of *Chlamydia pneumoniae,* the role of the immune defense in the physiology and pathology of the disease should probably be understood in order to develop satisfactory vaccines.

More detailed information relating to the biology of these strains, their interactions with their hosts, the associated phenomena of infectivity and those of escaping the immune defenses of the host in particular, and finally their involvement in the development of the these associated pathologies, will allow a better understanding of these mechanisms. In the light of the preceding text which shows in particular the limitations of the means of controlling *Chlamydia pneumoniae* infection, it is therefore at present essential, on the one hand, to develop molecular tools, in particular from a better genetic knowledge of *Chlamydia pneumoniae,* but also to develop new preventive and therapeutic treatments, new diagnostic methods and new vaccine strategies which are specific, effective and tolerated. This is precisely the object of the present invention.

The subject of the present invention is the nucleotide sequence ORF 291 encoding the amino acid sequence SEQ ID No.291 of the *Chlamydia pneumoniae* genome.

Thus, the subject of the present invention encompasses an isolated polynucleotide of an ORF sequence of a *pneumoniae* genome comprises:
a) the nucleotide sequence ORF 291 encoding the amino acid sequence SEQ ID No. 291 a nucleotide sequence exhibiting at least 80% identity with the sequence ORF 291.
b) a complementary sequence thereof.

Nucleotide sequence, polynucleotide or nucleic acid are understood to mean, according to the present invention, either a double-stranded DNA, a single-stranded DNA or products of transcription of the said DNAs.

It should be understood that the present invention does not relate to the genomic nucleotide sequences of *Chlamydia pneumoniae* taken in their natural environment, that is to say in the natural state. They are sequences which may have been isolated, purified or partially purified, by separation methods such as, for example, ion-exchange chromatography, molecular size exclusion chromatography or affinity chromatography, or alternatively fractionation techniques based on solubility in various solvents, or by genetic engineering methods such as amplification, cloning or subcloning, it being possible for the sequences of the invention to be carried by vectors.

The nucleotide sequence SEQ ID No. 1, published in the patent patent application WO 99/27105 or EP1032674, was obtained by sequencing the *Chlamydia pneumoniae* genome by the method of directed sequencing after fluorescent automated sequencing of the inserts of clones and assembling of these sequences of nucleotide fragments (inserts) by means of softwares (cf. Examples). In spite of the high precision of the sequence SEQ ID No. 1, it is possible that it does not perfectly, 100% represent the nucleotide sequence of the *Chlamydia pneumoniae* genome and that a few rare sequencing errors or uncertainties still remain in the sequence SEQ ID No. 1. In the present invention, the presence of an uncertainty for an amino acid is designated by "Xaa" and that for a nucleotide is designated by "N" in the sequence listing below. These few rare errors or uncertainties could be easily detected and corrected by persons skilled in the art using the entire chromosome and/or its representative fragments according to the invention and standard amplification, cloning and sequencing methods, it being possible for the sequences obtained to be easily compared, in particular by means of a computer software and using computer-readable media for recording the sequences according to the invention as described, for example, below. After correcting these possible rare errors or uncertainties, the corrected nucleotide sequence obtained would still exhibit at least 99.9% identity with the sequence SEQ ID No. 1. Such rare sequencing uncertainties are not present within the DNA contained within ATCC Deposit No.1366-VR, and whatever rare sequence uncertainties that exist within SEQ ID No. 1 can routinely be corrected utilizing the DNA of the ATCC deposits.

Homologous nucleotide sequence for the purposes of the present invention is understood to mean a nucleotide sequence having a percentage identity with the bases of the nucleotide sequence SEQ ID No. 1 of at least 80%, preferably 90% and 95%, this percentage being purely statistical and it being possible for the differences between the two nucleotide sequences to be distributed randomly and over their entire length. The said homologous sequences exhibiting a percentage identity with the bases of the nucleotide sequence SEQ ID No. 1 of at least 80%, preferably 90% and 95%, may comprise, for example, the sequences corresponding to the genomic sequence or to the sequences of its representative fragments of a bacterium belonging to the Chlamydia family, including the species *Chlamydia trachomatis, Chlamydia psittaci* and *Chlamydia pecorum* mentioned above, as well as the sequences corresponding to the genomic sequence or to the sequences of its representative fragments of a bacterium belonging to the variants of the species *Chlamydia pneumoniae.* In the present invention, the terms family and genus are mutually interchangeable, the terms variant, serotype, strain and subspecies are also mutually interchangeable. These homologous sequences may thus correspond to variations linked to mutations within the same species or between species and may correspond in particular to truncations, substitutions, deletions and/or additions of at least one nucleotide. The said homologous sequences may also correspond to variations linked to the degeneracy of the genetic code or to a bias in the genetic code which is specific to the family, to the species or to the variant and which are likely to be present in *Chlamydia.*

Protein and/or nucleic acid sequence homologies may be evaluated using any of the variety of sequence comparison algorithms and programs known in the art. Such algorithms and programs include, but are by no means limited to, TBLASTN, BLASTP, FASTA, TFASTA, and CLUSTALW (Pearson and Lipman, 1988, Proc. Natl. Acad Sci. USA 85(8):2444-2448; Altschul et al., 1990, J. Mol. Biol. 215(3):403-410; Thompson et al., 1994, Nucleic Acids Res. 22(2):4673-4680; Higgins et al., 1996, Methods Enzymol. 266:383-402; Altschul et al., 1990, J. Mol. Biol. 215(3):403-410; Altschul et al., 1993, Nature Genetics 3:266-272).

In a particularly preferred embodiment, protein and nucleic acid sequence homologies are evaluated using the Basic Local Alignment Search Tool ("BLAST") which is well known in the art (see, *e.g*., Karlin and Altschul, 1990, Proc. Natl. Acad. Sci. USA 87:2267-2268; Altschul et al., 1990, J. Mol. Biol. 215:403-410; Altschul et al., 1993, Nature Genetics 3:266-272; Altschul et al., 1997, Nuc. Acids Res. 25:3389-3402). In particular, five specific BLAST programs are used to perform the following task:
(1)BLASTP and BLAST3 compare an amino acid query sequence against a protein sequence database;
(2)BLASTN compares a nucleotide query sequence against a nucleotide sequence database;
(3)BLASTX compares the six-frame conceptual translation products of a query nucleotide sequence (both strands) against a protein sequence database;
(4)TBLASTN compares a query protein sequence against a nucleotide sequence database translated in all six reading frames (both strands); and
(5)TBLASTX compares the six-frame translations of a nucleotide query sequence against the six-frame translations of a nucleotide sequence database.

The BLAST programs identify homologous sequences by identifying similar segments, which are referred to herein as "high-scoring segment pairs," between a query amino or nucleic acid sequence and a test sequence which is preferably obtained from a protein or nucleic acid sequence database. High-scoring segment pairs are preferably identified (*i.e.,* aligned) by means of a scoring matrix, many of which are known in the art. Preferably, the scoring matrix used is the BLOSUM62 matrix (Gonnet et al., 1992, Science 256:1443-1445; Henikoff and Henikoff, 1993, Proteins 17:49-61). Less preferably, the PAM or PAM250 matrices may also be used (see, *e.g*., Schwartz and Dayhoff, eds., 1978, *Matrices for Detecting Distance Relationships: Atlas of Protein Sequence and Structure,* Washington: National Biomedical Research Foundation)

The BLAST programs evaluate the statistical significance of all high-scoring segment pairs identified, and preferably selects those segments which satisfy a user-specified threshold of significance, such as a user-specified percent homology. Preferably, the statistical significance of a high-scoring segment pair is evaluated using the statistical significance formula of Karlin (see, *e.g.,* Karlin and Altschul, 1990, Proc. Natl. Acad. Sci. USA 87:2267-2268).

Nucleotide sequence complementary to a sequence of the invention is understood to mean any DNA whose nucleotides are complementary to those of the sequence of the invention, and whose orientation is reversed (antiparallel sequence).

The ORF 291 nucleotide sequence is defined in Tables 1 and 2, *infra,* by its position on the sequence SEQ ID No. 1. ORF 291 has been identified via homology analyses as well as via analyses of potential ORF start sites, as discussed in the examples below. It is to be understood that the identified ORF of the invention comprises a nucleotide sequence that spans the contiguous nucleotide sequence from the ORF stop codon immediately 3' to the stop codon of the preceding ORF and through the 5' codon to the next stop codon of SEQ ID No.:1 in-frame to the ORF nucleotide sequence. Table 2, *infra,* lists the beginning, end and potential start site of ORF 291. In one embodiment, the ORF comprises the contiguous nucleotide sequence spanning from the potential ORF start site downstream (that is, 3') to the ORF stop codon (or the ORF codon immediately adjacent to and upstream of the ORF stop codon). ORF 291 encodes the polypeptide of SEQ ID No. 291. Table 1 also depicts the results of homology searches that compared the sequences of the polypeptides encoded by each of the ORFs to sequences present in public published databases.

By way of example and not limitation, procedures using conditions of high stringency are as follows: Prehybridization of filters containing DNA is carried out for 8 h to overnight at 65EC in buffer composed of 6X SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 h at 65EC, the preferred hybridization temperature, in prehybridization mixture containing 100 µg/ml denatured salmon sperm DNA and 5-20 X 10⁶ cpm of ³²P-labeled probe. Alternatively, the hybridization step can be performed at 65EC in the presence of SSC buffer, 1 x SSC corresponding to 0.15M NaCl and 0.05 M Na citrate. Subsequently, filter washes can be done at 37EC for 1 h in a solution containing 2X SSC, 0.01% PVP, 0.01% Ficoll, and 0.01% BSA, followed by a wash in 0.1X SSC at 50EC for 45 min. Alternatively, filter washes can be performed in a solution containing 2 x SSC and 0.1 % SDS, or 0.5 x SSC and 0.1% SDS, or 0.1 x SSC and 0.1% SDS at 68EC for 15 minute intervals. Following the wash steps, the hybridized probes are detectable by autoradiography. Other conditions of high stringency which may be used are well known in the art and as cited in Sambrook et al., 1989, Molecular Cloning, A Laboratory Manual, Second Edition, Cold Spring Harbor Press, N.Y., pp. 9.47-9.57; and Ausubel et al., 1989, Current Protocols in Molecular Biology, Green Publishing Associates and Wiley Interscience, N.Y. are incorporated herein in their entirety. Preferably, such sequences encode a homolog of a polypeptide encoded by one of ORF2 to ORF1297. In one embodiment, such sequences encode a *Chlamydia pneumoniae* polypeptide.

By way of example and not limitation, procedures using conditions of intermediate stringency are as follows: Filters containing DNA are prehybridized, and then hybridized at a temperature of 60EC in the presence of a 5 x SSC buffer and labeled probe. Subsequently, filters washes are performed in a solution containing 2x SSC at 50EC and the hybridized probes are detectable by autoradiography. Other conditions of intermediate stringency which may be used are well known in the art and as cited in Sambrook et al., 1989, Molecular Cloning, A Laboratory Manual, Second Edition, Cold Spring Harbor Press, N.Y., pp. 9.47-9.57; and Ausubel et al., 1989, Current Protocols in Molecular Biology, Green Publishing Associates and Wiley Interscience, N.Y. are incorporated herein in their entirety. Preferably, such sequences encode a homolog of a polypeptide encoded by one of ORF2 to ORF1297. In one embodiment, such sequences encode a *Chlamydia pneumoniae* polypeptide.

As regards the homology with the ORF 291 nucleotide sequence, the homologous sequences exhibiting a percentage identity with the bases of ORF 291 nucleotide sequences of at least 80%, preferably 90% and 95%, are preferred. Such homologous sequences are identified routinely via, for example, the algorithms described above and in the examples below. The said homologous sequences correspond to the homologous sequences as defined above and may comprise, for example, the sequences corresponding to the ORF sequences of a bacterium belonging to the Chlamydia family, including the species *Chlamydia trachomatis. Chlamydia psittaci* and *Chlarrrydia pecorum* mentioned above, as well as the sequences corresponding to the ORF sequences of a bacterium belonging to the variants of the species *Chlamydia pneumoniae.* These homologous sequences may likewise correspond to variations linked to mutations within the same species or between species and may correspond in particular to truncations, substitutions, deletions and/or additions of at least one nucleotides. The said homologous sequences may also correspond to variations linked to the degeneracy of the genetic code or to a bias in the genetic code which is specific to the family, to the species or to the variant and which are likely to be present in *Chlamydia.*

The invention comprises a polypeptide encoded by a nucleotide sequence according to the, invention, an ORF sequence, in particular the *Chlamydia pneumoniae* Polypeptides, characterized in that they are having the sequence SEQ ID No 291.

In the present description, the terms polypeptide, peptide and protein are interchangeable.

It should be understood that the invention does not relate to the polypeptides in natural form that is to say that they are not taken in their natural environment but that they may have been isolated or obtained by purification from natural sources, or alternatively obtained by genetic recombination, or else by chemical synthesis and that they may, in this case, comprise nonnatural amino acids, as will be described below.

Homologous polypeptide will be understood to designate the polypeptides exhibiting, in relation to the natural polypeptide, certain modifications such as in particular a deletion, addition or substitution of at least one amino acid, a truncation, an extension, a chimeric fusion, and/or a mutation, or polypeptides exhibiting post-translational modifications. Among the homologous polypeptides, those whose amino acid sequence exhibits at least 80%, preferably 90%, homology or identity with the amino acid sequences of the polypeptides according to the disclosure can be cited. In the case of a substitution, one or more consecutive or nonconsecutive amino acids are replaced by "equivalent" amino acids. The expression "equivalent" amino acid is intended here to designate any amino acid capable of being substituted for one of the amino acids in the basic structure without, however, essentially modifying the biological activities of the corresponding peptides and as will be defined later.

A polypeptide fragment according to the invention is understood to designate a polypeptide comprising a minimum of 5 amino acids, preferably 10 amino acids or preferably 15 amino acids. It is to be understood that such fragments refer only to portions of the polypeptide encoded by ORF 291 that are not currently listed in a publicly available database.

The polypeptide fragments according to the invention may correspond to isolated or purified fragments which are naturally present in *Chlamydia pneumoniae* or which are secreted by *Chlamydia pneumoniae,* or may correspond to fragments capable of being obtained by cleaving the said polypeptide with a proteolytic enzyme, such as trypsin or chymotrypsin or collagenase, or with a chemical reagent, such as cyanogen bromide (CNBr) or alternatively by placing the said polypeptide in a highly acidic environment, for example at pH 2.5. Such polypeptide fragments may be equally well prepared by chemical synthesis, using hosts transformed with an expression vector according to the invention containing a nucleic acid allowing the expression of the said fragments, placed under the control of appropriate elements for regulation and/or expression.

The structure of the cytoplasmic membranes and of the wall of bacteria is dependent on the associated proteins. The structure of the cytoplasmic membrane makes it impermeable to water, to water-soluble substances and to small-sized molecules (ions, small inorganic molecules, peptides or proteins). To enter into or to interfere with a cell or a bacterium, a ligand must establish a special relationship with a protein anchored in the cytoplasmic membrane (the receptor). These proteins which are anchored on the membrane play an important role in metabolism since they control the exchanges in the bacterium. These exchanges apply to molecules of interest for the bacterium (small molecules such as sugars and small peptides) as well as undesirable molecules for the bacterium such as antibiotics or heavy metals.

The double lipid layer structure of the membrane requires the proteins which are inserted therein to have hydrophobic domains of about twenty amino acids forming an alpha helix. Predominantly hydrophobic and potentially transmembrane regions may be predicted from the primary sequence of the proteins, itself deduced from the nucleotide sequence. The presence of one or more putative transmembrane domains raises the possibility for a protein to be associated with the cytoplasmic membrane and to be able to play an important metabolic role therein or alternatively for the protein thus exposed to be able to exhibit potentially protective epitopes.

If the proteins inserted into the membrane exhibit several transmembrane domains capable of interacting with one another via electrostatic bonds, it then becomes possible for these proteins to form pores which go across the membrane which becomes permeable for a number of substances. It should be noted that proteins which do not have transmembrane domains may also be anchored by the intermediacy of fatty acids in the cytoplasmic membrane, it being possible for the breaking of the bond between the protein and its anchor in some cases to be responsible for the release of the peptide outside the bacterium.

Also forming part of the invention are polypeptides encoded by the polynucleotides of the invention, as well as fusion polypeptides comprising such polypeptides. In one embodiment, the polypeptides and fusion polypeptides immunoreact with seropositive serum of an individual infected with *Chlamydia pneumoniae.* For example, described below, are polypeptide sequences exhibiting particularly preferable characteristics.

The subject of the invention is also a polypeptide according to the invention, characterized in that it is a polypeptide of the cellular envelope, preferably of the outer cellular envelope, of *Chlamydia pneumoniae* or one of its representative fragments. According to the invention, the said polypeptide is the polypeptide having the sequence SEQ ID No.291 and one of their representative fragments.

Preferably, the invention relates to a polypeptide according to the invention, characterized in that it is a *Chlamydia pneumoniae* transmembrane polypeptide or one of its representative fragments, having between 1 and 3 transmembrane domains, and in that it is the polypeptide having the sequence SEQ ID NO.291.

Preferably, the invention relates to a polypeptide according to the invention, in that it is a *Chlamydia pneumoniae* surface exposed polypeptide or one of its representative fragments, and in that it is the polypeptide having the sequence SEQ ID No.291 fragments.

Preferably, the invention relates to a polypeptide according to the invention, characterized in that it is a *Chlamydia pneumoniae* lipoprotein or one of its representative fragments, and in that it is the polypeptide having the sequence SEQ ID No.291.

Preferably, the invention relates to a polypeptide according to the invention, in that it is a *Chlamydia pneumoniae* polypeptide or one of its representative fragments not found in *Chlamydia trachomatis* (Blastp P>e⁻¹⁰), and in that it is the polypeptide having the sequence SEQ ID No.291.

In general, in the present invention, the functional group to which a polypeptide of the invention belongs, as well as its corresponding nucleotide sequence, may be determined either by comparative analogy with sequences already known, or by the use of standard techniques of biochemistry, of cytology combined with the techniques of genetic engineering such as immunoaffinity, localization by immunolabelling, differential extraction, measurement of enzymatic activity, study of the activity inducing or repressing expression or the study of expression in *E*. *coli.* It is also clearly understood that, in the present disclosure, a nucleotide sequence (ORF) or an amino acid sequence mentioned within a given functional group may also be part of another group taking into account, for example, the interrelationship between the groups listed. Accordingly, and as an example of this interrelationship, an exported and/or secreted polypeptide as well as its coding nucleotide sequence may also be involved in the *Chlamydia pneumoniae* virulence process by modifying the defense mechanism of the infected host cell, or a transmembrane polypeptide or its coding nucleotide sequence is also part of the polypeptides or coding nucleotide sequences of the cellular envelope.

The invention also discloses the nucleotide sequences according to the invention, characterized in that they are covalently or noncovalently immobilized on a support.

According to another advantageous embodiment of the nucleic sequences according to the disclosure, the latter may be used immobilized on a support and may thus serve to capture, through specific hybridization, the target nucleic acid obtained from the biological sample to be tested. If necessary, the solid support is separated from the sample and the hybridization complex formed between the so-called capture probe and the target nucleic acid is then detected by means of a second probe, called detection probe, labelled with an easily detectable element.

The nucleotide sequences according to the invention may also be used in new analytical systems, DNA chips, which allow sequencing, the study of mutations and of the expression of genes, and which are currently of interest given their very small size and their high capacity in terms of number of analyses.

The principle of the operation of these chips is based on molecular probes, most often oligonucleotides, which are attached onto a miniaturized surface, generally of the order of a few square centimetres. During an analysis, a sample containing fragments of a target nucleic acid to be analysed, for example DNA or RNA labelled, for example, after amplification, is deposited onto the DNA chip in which the support has been coated beforehand with probes. Bringing the labelled target sequences into contact with the probes leads to the formation, through hybridization, of a duplex according to the rule of pairing defined by J.D. Watson and F. Crick. After a washing step, analysis of the surface of the chip allows the effective hybridizations to be located by means of the signals emitted by the labels tagging the target. A hybridization fingerprint results from this analysis which, by appropriate computer processing, will make it possible to determine information such as the presence of specific fragments in the sample, the determination of sequences and the presence of mutations.

The chip consists of a multitude of molecular probes, precisely organized or arrayed on a solid support whose surface is miniaturized. It is at the centre of a system where other elements (imaging system, microcomputer) allow the acquisition and interpretation of a hybridization fingerprint.

The hybridization supports are provided in the form of flat or porous surfaces (pierced with wells) composed of various materials. The choice of a support is determined by its physicochemical properties, or more precisely, by the relationship between the latter and the conditions under which the support will be placed during the synthesis or the attachment of the probes or during the use of the chip. It is therefore necessary, before considering the use of a particular support (R.S. Matson et al., 1994), to consider characteristics such as its stability to pH, its physical strength, its reactivity and its chemical stability as well as its capacity to nonspecifically bind nucleic acids. Materials such as glass, silicon and polymers are commonly used. Their surface is, in a first step, called "functionalization", made reactive towards the groups which it is desired to attach thereon. After the functionalization, so-called spacer molecules are grafted onto the activated surface. Used as intermediates between the surface and the probe, these molecules of variable size render unimportant the surface properties of the supports, which often prove to be problematic for the synthesis or the attachment of the probes and for the hybridization.

Among the hybridization supports, there may be mentioned glass which is used, for example, in the method of in situ synthesis of oligonucleotides by photochemical addressing developed by the company Affymetrix (E.L. Sheldon, 1993), the glass surface being activated by silane. Genosensor Consortium (P. Mérel, 1994) also uses glass slides carrying wells 3 mm apart, this support being activated with epoxysilane.

Polymers or silicon may also be mentioned among these hybridization supports. For example, the Andrein Mirzabekov team has developed a chip consisting of polyacrylamide squares polymerized on a silanized glass surface (G. Yershov et al., 1996). Several teams use silicon, in particular the IFOS laboratory of Ecole Centrale of Lyon which uses a silicon semiconductor substrate which is p-doped by introducing it into its crystalline structure atoms whose valency is different from that of silicon. Various types of metals, in particular gold and platinum, may also be used as support (Genosensor Consortium (K. Beattie et al., 1993)).

The probes according to the invention may be synthesized directly in situ on the supports of the DNA chips. This in situ synthesis may be carried out by photochemical addressing (developed by the company Affymax (Amsterdam, Holland) and exploited industrially by its subsidiary Affymetrix (United States)) or based on the VLSIPS (very large scale immobilized polymer synthesis) technology (S.P.A. Fodor et al., 1991) which is based on a method of photochemically directed combinatory synthesis and the principle of which combines solid-phase chemistry, the use of photolabile protecting groups and photolithography.

The probes according to the invention may be attached to the DNA chips in various ways such as electrochemical addressing, automated addressing or the use of probe printers (T. Livache et al., 1994; G. Yershov et al., 1996; J. Derisi et al., 1996, and S. Borman, 1996).

The revealing of the hybridization between the probes of the invention, deposited or synthesized in situ on the supports of the DNA chips, and the sample to be analysed, may be determined, for example, by measurement of fluorescent signals, by radioactive counting or by electronic detection.

The use of fluorescent molecules such as fluorescein constitutes the most common method of labelling the samples. It allows direct or indirect revealing of the hybridization and allows the use of various fluorochromes.

Affymetrix currently provides an apparatus or a scanner designed to read its Gene Chip™ chips. It makes it possible to detect the hybridizations by scanning the surface of the chip in confocal microscopy (R.J. Lipshutz et al., 1995). Other methods of detecting fluorescent signals have been tested: coupling of an epifluorescence microscope and a CCD camera (G. Yershov et al., 1996), the use of an optical fibre collecting system (E.L. Sheldon, 1993). A conventional method consists in carrying out an end labelling, with phosphorus 32, of the target sequences, by means of an appropriate apparatus, the Phosphorimager (marketed by Molecular Dynamics). The electronic detection is based on the principle that the hybridization of two nucleic acid molecules is accompanied by physical phenomena which can be quantified under certain conditions (system developed by Ecole Centrale of Lyon and called GEN-FET (GEN field effect transistor)). Genosensor Consortium and the company Beckman Instruments who are developing an electronic chip or Permittivity Chips™ may also be mentioned (K. Beattie et al., 1993).

The nucleotide sequences according to the invention may thus be used in DNA chips to carry out the analysis of mutations. This analysis is based on the production of chips capable of analysing each base of a nucleotide sequence according to the invention.

The nucleotide sequences according to the invention may also be used in DNA chips to carry out the analysis of the expression of the *Chlamydia pneumoniae* genes. This analysis of the expression of *Chlamydia pneumoniae* genes is based on the use of chips where probes of the invention, chosen for their specificity to characterize a given gene, are present (D.J. Lockhart et al., 1996; D.D. Shoemaker et al., 1996). For the methods of analysis of gene expression using the DNA chips, reference may, for example, be made to the methods described by DJ. Lockhart et al. (1996) and Sosnowsky et al. (1997) for the synthesis of probes in situ or for the addressing and the attachment of previously synthesized probes. The target sequences to be analysed are labelled and in general fragmented into sequences of about 50 to 100 nucleotides before being hybridized onto the chip. After washing as described, for example, by DJ. Lockhart et al. (1996) and application of different electric fields (Sosnowsky et al., 1997), the labelled compounds are detected and quantified, the hybridizations being carried out at least in duplicate. Comparative analyses of the signal intensities obtained with respect to the same probe for different samples and/or for different probes with the same sample, determine the differential expression of RNA or of DNA derived from the sample.

The nucleotide sequences according to the invention may, in addition, be used in DNA chips where other nucleotide probes specific for other microorganisms are also present, and may allow the carrying out of a serial test allowing rapid identification of the presence of a microorganism in a sample.

Accordingly, the subject of the disclosure is also the nucleotide sequences according to the invention, characterized in that they are immobilized on a support of a DNA chip.

The DNA chips, characterized in that they contain at least one nucleotide sequence according to the invention, immobilized on the support of the said chip, also form part of the invention.

The said chips will preferably contain several probes or nucleotide sequences of the invention of different length and/or corresponding to different genes so as to identify, with greater certainty, the specificity of the target sequences or the desired mutation in the sample to be analysed.

Accordingly, the analyses carried out by means of primers and/or probes according to the invention, immobilized on supports such as DNA chips, will make it possible, for example, to identify, in samples, mutations linked to variations such as intraspecies variations. These variations may be correlated or associated with pathologies specific to the variant identified and will make it possible to select the appropriate treatment.

Another subject of the present invention is a vector for the cloning and/or the expression of a sequence, characterized in that it contains a nucleotide sequence according to the invention.

According to the invention, the vectors comprise the elements necessary to allow the expression and/or the secretion of the said nucleotide sequences in a given host cell, and form part of the invention. The vector should, in this case, comprise a promoter, signals for initiation and for termination of translation, as well as appropriate regions for regulation of transcription. It should be capable of being stably maintained in the host cell and may optionally possess particular signals specifying the secretion of the translated protein. These different elements are chosen according to the host cell used. To this effect, the nucleotide sequences according to the invention may be inserted into autonomously-replicating vectors within the chosen host, or integrative vectors in the chosen host.

Any of the standard methods known to those skilled in the art for the insertion of DNA fragments into a vector may be used to construct expression vectors containing a chimeric gene consisting of appropriate transcriptional/translational control signals and the protein coding sequences. These methods may include *in vitro* recombinant DNA and synthetic techniques and in vivo recombinants (genetic recombination).

Expression of a polypeptide, peptide or derivative, or analogs thereof encoded by a polynucleotide sequence ORF 291 contained within SEQ ID No. 1 may be regulated by a second nucleic acid sequence so that the protein or peptide is expressed in a host transformed with the recombinant DNA molecule. For example, expression of a protein or peptide may be controlled by any promoter/enhancer element known in the art. Promoters which may be used to control expression include, but are not limited to, the CMV promoter, the SV40 early promoter region (Bernoist and Chambon, 1981, Nature 290:304-310), the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42); prokaryotic, expression vectors such as the 3-lactamase promoter (Villa-Kamaroff, et al., 1978, Proc. Natl. Acad. Sci. U.S.A. 75:3727-3731), or the tac promoter (DeBoer, et al., 1983, Proc. Natl. Acad. Sci. U.S.A. 80:21-25); see also "Useful proteins from recombinant bacteria" in Scientific American, 1980, 242:74-94; plant expression vectors comprising the nopaline synthetase promoter region (Herrera-Estrella et al., 1983, Nature 303:209-213) or the cauliflower mosaic virus 35S RNA promoter (Gardner, et al., 1981, Nucl. Acids Res. 9:2871), and the promoter of the photosynthetic enzyme ribulose biphosphate carboxylase (Herrera-Estrella et al., 1984, Nature 310:115-120); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58; alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94; myelin basic protein gene control region which is active in oligodendrocyte cells in the brain (Readhead et al., 1987, Cell 48:703-712); myosin light chain-2 gene control region which is active in skeletal muscle (Sani, 1985, Nature 314:283-286), and gonadotropic releasing hormone gene control region which is active in the hypothalamus (Mason et al., 1986, Science 234:1372-1378).

The vectors according to the invention are, for example, vectors of plasmid or viral origin. In a specific embodiment, a vector is used that comprises a promoter operably linked to a protein or peptide-encoding a nucleic acid sequence ORF 291 contained within SEQ ID No. 1, one or more origins of replication, and, optionally, one or more selectable markers (*e.g*., an antibiotic resistance gene). Expression vectors comprise regulatory sequences that control gene expression, including gene expression in a desired host cell. Preferred vectors for the expression of the polypeptides of the invention include the pET-type plasmid vectors (Promega) or pBAD plasmid vectors (Invitrogen). Furthermore, the vectors according to the invention are useful for transforming host cells so as to clone or express the nucleotide sequences of the invention.

Expression can also be achieved using targeted homologous recombination to activate *Chlamydia pneumoniae* genes present in the cloned genomic DNA. A heterologous regulatory element may be inserted into a stable cell line or cloned microorganism, such that it is operatively linked with an endogenous *Chlamydia pneumoniae* gene present in the cloned genome, using techniques, such as targeted homologous recombination, which are well known to those of skill in the art (*See, e.g.,* Chappel, U.S. Patent No. 4,215,051 and Skoultchi, WO 91/06667 each of which is incorporated herein in its entirety).

Expression vector/host cell systems containing inserts of polynucleotide sequences in SEQ ID No. 1 or ORFs within SEQ ID No. 1, which encode polypeptides, peptides or derivatives, or analogs thereof, can be identified by three general approaches: (a) nucleic acid hybridization, (b) presence or absence of "marker" gene functions, and (c) expression of inserted sequences. In the first approach, the presence of a polynucleotide sequence inserted in an expression vector can be detected by nucleic acid hybridization using probes comprising sequences that are homologous to an inserted polynucleotide sequence. In the second approach, the recombinant vector/host system can be identified and selected based upon the presence or absence of certain "marker" gene functions (*e.g*., thymidine kinase activity, resistance to antibiotics, transformation phenotype, occlusion body formation in baculovirus, etc.) caused by the insertion of a polynucleotide sequence in the vector. For example, if the polynucleotide sequence in SEQ ID No. 1 or ORFs within SEQ ID No. 1 is inserted within the marker gene sequence of the vector, recombinants containing the insert can be identified by the absence of the marker gene function. In the third approach, recombinant expression vectors can be identified by assaying the product of the polynucleotide sequence expressed by the recombinant. Such assays can be based, for example, on the physical or functional properties of the expressed polypeptide in *in vitro* assay systems, *e.g*., binding with antibody, promotion of cell proliferation.

Once a particular recombinant DNA molecule is identified and isolated, several methods known in the art may be used to propagate it. The clones identified may be introduced into an appropriate host cell by standard methods, such as for example lipofection, electroporation, and heat shock. Once a suitable host system and growth conditions are established, recombinant expression vectors can be propagated and prepared in quantity.

The invention also encompasses the host cells transformed by a vector according to the invention. These cells may be obtained by introducing into host cells a nucleotide sequence inserted into a vector as defined above, and then culturing the said cells under conditions allowing the replication and/or the expression of the transfected nucleotide sequence.

The host cell may be chosen from eukaryotic or prokaryotic systems, such as for example bacterial cells (Olins and Lee, 1993), but also yeast cells (Buckholz, 1993), as well as animal cells, in particular cultures of mammalian cells (Edwards and Aruffo, 1993), and in particular Chinese hamster ovary (CHO) cells, but also insect cells in which methods using baculoviruses for example may be used (Luckow, 1993).

Furthermore, a host cell strain may be chosen which modulates the expression of the inserted sequences, or modifies and processes the gene product in the specific fashion desired. Expression from certain promoters can be elevated in the presence of certain inducers; thus, expression of the genetically engineered polypeptide may be controlled. Furthermore, different host cells have characteristic and specific mechanisms for the translational and post-translational processing and modification (*e.g*., glycosylation, phosphorylation) of proteins. Appropriate cell lines or host systems can be chosen to ensure the desired modification and processing of the foreign protein expressed. For example, expression in a bacterial system can be used to produce an unglycosylated core protein product. Expression in yeast will produce a glycosylated product. Expression in mammalian cells can be used to ensure "native" glycosylation of a heterologous protein. Furthermore, different vector/host expression systems may effect processing reactions to different extents.

A preferred host cell for the expression of the proteins of the invention consists of prokaryotic cells, such as Gram bacteria. A further preferred host cell according to the invention is a bacterium belonging to the *Chlamydia* family, more preferably belonging to the species *Chlamydia pneumoniae* or chosen from a microorganism associated with the species *Chlamydia pneumoniae.*

In other specific embodiments, the polypeptides, peptides or derivatives, or analogs thereof may be expressed as a fusion, or chimeric protein product (comprising the protein, fragment, analog, or derivative joined via a peptide bond to a heterologous protein sequence (of a different protein)). Such a chimeric product can be made by ligating the appropriate nucleic acid sequences encoding the desired amino acid sequences to each other by methods known in the art, in the proper coding frame, and expressing the chimeric product by methods commonly known in the art. Alternatively, such a chimeric product may be made by protein synthetic techniques, *e.g*., by use of a peptide synthesizer.

Genomic sequences can be cloned and expressed as translational gene products (*i.e*., peptides, polypeptides, and proteins) or transcriptional gene products (*i.e*., antisense and ribozymes).

It is now possible to produce recombinant polypeptides in a relatively large quantity by genetic engineering using the cells transformed with expression vectors according to the invention or using transgenic animals according to the invention.

The methods of preparing a polypeptide of the invention in recombinant form, characterized in that they use a vector and/or a cell transformed with a vector according to the invention and/or a transgenic animal comprising one of the said transformed cells according to the invention, are themselves included in the present invention.

Among the said methods of preparing a polypeptide of the invention in recombinant form, the methods of preparation using a vector, and/or a cell transformed with the said vector and/or a transgenic animal comprising one of the said transformed cells, containing a nucleotide sequence encoding a polypeptide of the cellular envelope of *Chlamydia pneumoniae* or one of its representative fragments, more preferably encoding a polypeptide of the outer cellular envelope of *Chlamydia pneumoniae* or one of its fragment, are preferred.

The recombinant polypeptides obtained as indicated above may be provided either in glycosylated or non-glycosylated form and may or may not have the natural tertiary structure.

A preferred variant consists in producing a recombinant polypeptide fused to a "carrier" protein (chimeric protein). The advantage of this system is that it allows a stabilization and a reduction in proteolysis of the recombinant product, an increase in solubility during renaturation in vitro and/or a simplification of purification when the fusion partner has affinity for a specific ligand.

More particularly, the invention relates to a method of preparing a polypeptide of the invention comprising the following steps:
a) culture of the transformed cells under conditions allowing the expression of a recombinant polypeptide having a nucleic acid sequence according to the invention;
b) where appropriate, recovery of the said recombinant polypeptide.

When the method of preparing a polypeptide of the invention uses a transgenic animal according to the invention, the recombinant polypeptide is then extracted from the said animal.

The subject of the invention is also a polypeptide capable of being obtained by a method of the invention as described above.

The invention also comprises a method of preparing a synthetic polypeptide, characterized in that it uses an amino acid sequence of polypeptides according to the invention.

The invention also relates to a synthetic polypeptide obtained by a method according to the invention.

Polypeptides according to the invention may also be prepared by conventional techniques in the field of peptide synthesis under conditions suitable to produce the polypeptides encoded by the polynucleotide of the invention. This synthesis may be carried out in and recovered from a homogeneous solution or on a solid phase.

For example, the synthesis technique in a homogeneous solution described by Houbenweyl in 1974 may be used.

This method of synthesis consists in successively condensing, in pairs, the successive amino acids in the required order, or in condensing amino acids and fragments previously formed and already containing several amino acids in the appropriate order, or alternatively several fragments thus previously prepared, it being understood that care will have been taken to protect beforehand all the reactive functional groups carried by these amino acids or fragments, with the exception of the amine functional groups of one and the carboxyl functional groups of the other or vice versa, which should normally take part in the formation of the peptide bonds, in particular after activation of the carboxyl functional group, according to methods well known in peptide synthesis.

According to another preferred technique of the invention, the one described by Merrifield is used.

To manufacture a peptide chain according to the Merrifield method, a highly porous polymer resin is used, onto which the first C-terminal amino acid of the chain is attached. This amino acid is attached onto a resin via its carboxyl group and its amine functional group is protected. The amino acids which will constitute the peptide chain are thus attached, one after another, onto the amine group, each time deprotected beforehand, of the portion of the peptide chain already formed, and which is attached to the resin. When the entire peptide chain desired is formed, the protecting groups are removed from the various amino acids constituting the peptide chain and the peptide is detached from the resin with the aid of an acid.

The invention relates, in addition, to hybrid (fusion) polypeptides having at least one polypeptide or one of its representative fragments according to the invention, and a sequence of a polypeptide capable of eliciting an immune response in humans or animals.

Advantageously, the antigenic determinant is such that it is capable of eliciting a humoral and/or cellular response. An antigenic determinant may be identified by screening expression libraries of the *Chlamydia pneumoniae* genome with antibodies contained in the serum of patients infected with a bacterium belonging to the species *Chlamydia pneumoniae.* An antigenic determinant may comprise a polypeptide or one of its representative fragments according to the invention, in glycosylated form, used in order to obtain immunogenic compositions capable of inducing the synthesis of antibodies directed against multiple epitopes. The said polypeptides or their glycosylated fragments also form part of the invention.

These hybrid molecules may consist, in part, of a carrier molecule for polypeptides or for their representative fragments according to the invention, combined with a portion which may be immunogenic, in particular an epitope of the diphtheria toxin, the tetanus toxin, a hepatitis B virus surface antigen (patent FR 79 21811), the poliomyelitis virus VP1 antigen or any other viral or bacterial toxin or antigen.

The methods of synthesizing the hybrid molecules include the methods used in genetic engineering to construct hybrid nucleotide sequences encoding the desired polypeptide sequences. Reference may be advantageously made, for example, to the technique for producing genes encoding fusion proteins described by Minton in 1984.

The said hybrid nucleotide sequences encoding a hybrid polypeptide as well as the hybrid polypeptides according to the invention, characterized in that they are recombinant polypeptides obtained by the expression of the said hybrid nucleotide sequences, also form part of the invention.

The invention also comprises the vectors characterized in that they contain one of the said hybrid nucleotide sequences. The host cells transformed by the said vectors, the transgenic animals comprising one of the said transformed cells as well as the methods of preparing recombinant polypeptides using the said vectors, the said transformed cells and/or the said transgenic animals of course also form part of the invention.

The polypeptides according to the invention, the antibodies according to the invention described below and the nucleotide sequences according to the invention may advantageously be used in *in vitro* and/or *in vivo* methods for the detection and/or the identification of bacteria belonging to the species *Chlamydia pneumoniae,* in a biological sample (biological tissue or fluid) which is likely to contain them. These methods, depending on the specificity of the polypeptides, of the antibodies and of the nucleotide sequences according to the invention which will be used, may in particular detect and/or identify the bacterial variants belonging to the species *Chlamydia pneumoniae* as well as the associated microorganisms capable of being detected by the polypeptides, the antibodies and the nucleotide sequences according to the invention which will be chosen. It may, for example, be advantageous to choose a polypeptide, an antibody or a nucleotide sequence according to the invention, which is capable of detecting any bacterium of the Chlamydia family by choosing a polypeptide, an antibody and/or a nucleotide sequence according to the invention which is specific to the family or, on the contrary, it will be most particularly advantageous to target a variant of the species *Chlamydia pneumoniae,* which is responsible, for example, for the induction or the worsening of pathologies specific to the targeted variant, by choosing a polypeptide, an antibody and/or a nucleotide sequence according to the invention which is specific to the said variant.

The polypeptides according to the invention may advantageously be used in a method for the detection and/or the identification of bacteria belonging to the species *Chlamydia pneumoniae* or to an associated microorganism, in a biological sample (biological tissue or fluid) which is likely to contain them, characterized in that it comprises the following steps:
a) bringing this biological sample into contact with a polypeptide or one of its representative fragments according to the invention (under conditions allowing an immunological reaction between the said polypeptide and the antibodies which may be present in the biological sample);
b) detecting the antigen-antibody complexes which may be formed.

Preferably, the biological sample consists of a fluid, for example a human or animal serum, blood or biopsies.

Any conventional procedure may be used to carry out such a detection of the antigen-antibody complexes which may be formed.

By way of example, a preferred method uses immunoenzymatic procedures based on the ELISA technique, immunofluorescence procedures or radioimmunological procedures (RIA), and the like.

Accordingly, the invention also relates to the polypeptides according to the invention, labelled with the aid of a suitable label such as a label of the enzymatic, fluorescent or radioactive type.

Such methods comprise, for example, the following steps:
- deposition of defined quantities of a polypeptide composition according to the invention into the wells of a microtitre plate,
- introduction, into the said wells, of increasing dilutions of serum, or of a different biological sample as defined above, which has to be analysed,
- incubation of the microplate,
- introduction, into the wells of the microtitre plate, of labelled antibodies directed against human or animal immunoglobulins, these antibodies having been labelled with the aid of an enzyme selected from those which are capable of hydrolyzing a substrate, thereby modifying the absorption of the radiation of the latter, at least at a defined wavelength, for example at 550 nm,
- detection, by comparison with a control, of the quantity of substrate hydrolyzed.

The invention also relates to a kit or set for the detection and/or the identification of bacteria belonging to the species *Chlamydia pneumoniae* or to an associated microorganism, characterized in that it comprises the following components:
- a polypeptide according to the invention,
- where appropriate, the reagents for constituting the medium appropriate for the immunological or specific reaction,
- the reagents allowing the detection of the antigen-antibody complexes produced by the immunological reaction between the polypeptide(s) of the invention and the antibodies which may be present in the biological sample, it being possible for these reagents also to carry a label, or to be capable of being recognized in turn by a labelled reagent, more particularly in the case where the polypeptide according to the invention is not labelled,
- where appropriate, a reference biological sample (negative control) free of antibodies recognized by a polypeptide according to the invention,
- where appropriate, a reference biological sample (positive control) containing a predetermined quantity of antibodies recognized by a polypeptide according to the invention.

According to the invention, the polypeptides, peptides, fusion proteins or other derivatives, or analogs thereof encoded by a polynucleotide sequence ORF 291, may be used as an immunogen to generate antibodies which immunospecifically bind such an immunogen. Such antibodies may include, but are not limited to, polyclonal and monoclonal antibodies, humanized or chimeric antibodies, single chain antibodies, Fab fragments, F(ab')₂ fragments, fragments produced by a Fab expression library, anti-idiotypic (anti-Id) antibodies, and epitope-binding fragments of any of the above. In a specific embodiment, the antibody to a polypeptide, peptide or other derivative, or analog thereof encoded by a polynucleotide sequence ORF 291 is a bispecific antibody (see generally, *e.g*. Fanger and Drakeman, 1995, Drug News and Perspectives 8; 133-137). Such a bispecific antibody is genetically engineered to recognize both (1) an epitope and (2) one of a variety of "trigger" molecules, *e.g*. Fc receptors on myeloid cells, and CD3 and CD2 on T cells, that have been identified as being able to cause a cytotoxic T-cell to destroy a particular target. Such bispecific antibodies can be prepared either by chemical conjugation, hybridoma, or recombinant molecular biology techniques known to the skilled artisan.

Various procedures known in the art may be used for the production of polyclonal antibodies to a polypeptide, peptide or other derivative, or analog thereof encoded by a polynucleotide sequence in SEQ ID No.1. For the production of antibody, various host animals can be immunized by injection with a polypeptide, or peptide or other derivative, or analog thereof, including but not limited to rabbits, mice, rats, etc. Various adjuvants, depending on the host species, may be used to increase the immunological response, including but not limited to Stimulon™ QS-21 (Aquila Biopharmaceuticals, Inc., Framingham, MA), MPL™ (3-O-deacylated monophosphoryl lipid A; RIBI ImmunoChem Research, Inc., Hamilton, MT), aluminum phosphate, IL-12 (Genetics Institute, Cambridge, MA), Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, BCG (bacille Calmette-Guerin), and corynebacterium parvum. Alternatively, polyclonal antibodies may be prepared by purifying, on an affinity column onto which a polypeptide according to the invention has been previously attached, the antibodies contained in the serum of patients infected with a bacterium belonging to the species *Chlamydia pneumoniae.*

For preparation of monoclonal antibodies directed toward a polypeptide, peptide or other derivative, or analog, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique-to produce human monoclonal antibodies (Cole et al., 1985, in Monoclonal Antibodies and Cancer Therapy, Alan R. Liss, Inc., pp. 77-96). In an additional embodiment of the invention, monoclonal antibodies can be produced in germ-free animals utilizing technology described in PCT/US90/02545. In another embodiment of the invention, transgenic non-human animals can be used for the production of human antibodies utilizing technology described in WO 98/24893 and WO 96/33735. According to the invention, human antibodies may be used and can be obtained by using human hybridomas (Cote et al., 1983, Proc. Natl. Acad. Sci. U.S.A. 80:2026-2030) or by transforming human B cells with EBV virus *in vitro* (Cole et al., 1985, in Monoclonal Antibodies and Cancer Therapy, Alan R. Liss, pp. 77-96). In fact, according to the invention, techniques developed for the production of "chimeric antibodies" (Morrison et al., 1984, PROC. NATL. ACAD. SCI. U.S.A. 81:6851-6855; Neuberger et al., 1984, Nature 312:604-608; Takeda et al., 1985, Nature 314:452-454) by splicing the genes from a mouse antibody molecule specific for a polypeptide, peptide or other derivative, or analog together with genes from a human antibody molecule of appropriate biological activity can be used; such antibodies are within the scope of this invention.

According to the invention, techniques described for the production of single chain antibodies (U.S. Patent 4,946,778) can be adapted to produce polypeptide or peptide-specific single chain antibodies. An additional embodiment of the invention utilizes the techniques described for the construction of Fab expression libraries (Huse et al., 1989, Science 246:1275-1281) to allow rapid and easy identification of monoclonal Fab fragments with the desired specificity for polypeptides, derivatives, or analogs.

Antibody fragments which contain the idiotype of the molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')₂ fragment, the Fab fragments which can be generated by treating the antibody molecule with papain and a reducing agent, and Fv fragments.

In addition, techniques have been developed for the production of chimerized (*See,* e.g., Boss, M. et al., U.S. Patent No. 4,816,397; and Cabilly, S. et al., U.S. Patent No. 5,585,089 each of which is incorporated herein by reference in its entirety) humanized antibodies (*See,* e.g., Queen, U.S. Patent No. 5,585,089, which is incorporated herein by reference in its entirety.) An immunoglobulin light or heavy chain variable region consists of a "framework" region interrupted by three hypervariable regions, referred to as complementarily determining regions (CDRs). The extent of the framework region and CDRs have been precisely defined ( See, "Sequences of Proteins of Immunological Interest", Kabat, E. et al., U.S. Department of Health and Human Services (1983). Briefly, humanized antibodies are antibody molecules from non-human species having one or more CDRs from the non-human species and a framework from a human immunoglobulin molecule.

The antibodies of the invention may also be labelled in the same manner as described above for the nucleic probes of the invention such as an enzymatic, fluorescent or radioactive type labelling.

The invention relates, in addition, to a method for the detection and/or the identification of bacteria belonging to the species *Chlamydia pneumoniae* or to an associated microorganism in a biological sample, characterized in that it comprises the following steps:
a) bringing the biological sample (biological tissue or fluid) into contact with a mono- or polyclonal antibody according to the invention (under conditions allowing an immunological reaction between the said antibodies and the polypeptides of the bacterium belonging to the species *Chlamydia pneumoniae* or to an associated microorganism which may be present in the biological sample, that is, under conditions suitable for the formation of immune complexes);
b) detecting the antigen-antibody complex which may be formed.

Also falling within the scope of the invention is a kit or set for the detection and/or the identification of bacteria belonging to the species *Chlamydia pneumoniae* or to an associated microorganism, characterized in that it comprises the following components:
- a polyclonal or monoclonal antibody according to the invention, labeled where appropriate;
- where appropriate, a reagent for constituting the medium appropriate for carrying out the immunological reaction;
- a reagent allowing the detection of the antigen-antibody complexes produced by the immunological reaction, it being possible for this reagent also to carry a label, or to be capable of being recognized in turn by a labelled reagent, more particularly in the case where the said monoclonal or polyclonal antibody is not labelled;
- where appropriate, reagents for carrying out the lysis of the cells in the sample tested.

The principle of the DNA chip which was explained above may also be used to produce protein "chips" on which the support has been coated with a polypeptide or an antibody according to the invention, or arrays thereof, in place of the DNA. These protein "chips" make it possible, for example, to analyze the biomolecular interactions (BIA) induced by the affinity capture of target analytes onto a support coated, for example, with proteins, by surface plasma resonance (SPR). Reference may be made, for example, to the techniques for coupling proteins onto a solid support which are described in EP 524 800 or to the methods describing the use of biosensor-type protein chips such as the BIAcore-type technique (Pharmacia) (Arlinghaus et al., 1997, Krone et al., 1997, Chatelier et al., 1995). These polypeptides or antibodies according to the invention, capable of specifically binding antibodies or polypeptides derived from the sample to be analysed, may thus be used in protein chips for the detection and/or the identification of proteins in samples. The said protein chips may in particular be used for infectious diagnosis and may preferably contain, per chip, several polypeptides and/or antibodies of the invention of different specificity, and/or polypeptides and/or antibodies capable of recognizing microorganisms different from *Chlamydia pneumoniae.*

Accordingly, the subject of the present disclosure is also the polypeptides and the antibodies according to the invention, characterized in that they are immobilized on a support, in particular of a protein chip.

The protein chips, characterized in that they contain at least one polypeptide or one antibody according to the invention immobilized on the support of the said chip, also form part of the invention.

The present invention comprises, in addition, a protein chip according to the invention, characterized in that it contains, in addition, at least one polypeptide of a microorganism different from *Chlamydia pneumoniae* or at least one antibody directed against a compound of a microorganism different from *Chlamydia pneumoniae,* immobilized on the support of the said chip.

The disclosure also relates to a kit or set for the detection and/or the identification of bacteria belonging to the species *Chlamydia pneumoniae* or to an associated microorganism, or for the detection and/or the identification of a microorganism characterized in that it comprises a protein chip according to the invention.

The subject of the present disclosure is also a method for the detection and/or the identification of bacteria belonging to the species *Chlamydia pneumonia* or to an associated microorganism in a biological sample, characterized in that it uses a nucleotide sequence according to the invention.

More particularly, the invention relates to a method for the detection and/or the identification of bacteria belonging to the species *Chlamydia pneumoniae* in a biological sample, characterized in that it comprises the following steps:
a) where appropriate, isolation of the DNA from the biological sample to be analysed, or optionally production of a cDNA from the RNA in the biological sample;
b) specific amplification of the DNA of bacteria belonging to the species *Chlamydia pneumoniae* or to an associated microorganism with the aid of at least one primer which hybridizes specifically with the polynucleotide ORF 291.
c) detection of the amplification products.

These may be detected, for example, by the molecular hybridization technique using a nucleic probe according to the invention. This probe will be advantageously labelled with a nonradioactive (cold probe) or radioactive element.

For the purposes of the present invention, "DNA in the biological sample" or "DNA contained in the biological sample" will be understood to mean either the DNA present in the biological sample considered, or optionally the cDNA obtained after the action of a reverse transcriptase-type enzyme on the RNA present in the said biological sample.

Another aim of the present invention consists in a method according to the invention, characterized in that it comprises the following steps:
a) bringing a nucleotide probe according to the invention into contact with a biological sample, the DNA contained in the biological sample having, where appropriate, been previously made accessible to hybridization, under conditions allowing the hybridization of the probe to complementary base pairs of the DNA of a bacterium belonging to the species *Chlamydia pneumonia* or to an associated microorganism;
b) detecting the hybridization complex formed between the nucleotide probe and the DNA in the biological sample.

The present disclosure also relates to a method according to the invention, characterized in that it comprises the following steps:
a) bringing a nucleotide probe immobilized on a support according to the invention into contact with a biological sample, the DNA in the sample having, where appropriate, been previously made accessible to hybridization, under conditions allowing the hybridization of the probe to the DNA of a bacterium belonging to the species *Chlamydia pneumoniae* or to an associated microorganism;
b) bringing the hybrid formed between the nucleotide probe immobilized on a support and the DNA contained in the biological sample, were appropriate after removal of the DNA in the biological sample which has not hybridized with the probe, into contact with a labelled nucleotide probe according to the invention;
c) detecting the new hybrid formed in step b).

According to an advantageous embodiment of the method for the detection and/or the identification defined above, it is characterized in that, prior to step a), the DNA in the biological sample is primer-extended and/or amplified beforehand with the aid of at least one primer according to the invention.

The invention relates, in addition, to a kit or set for the detection and/or the identification of bacteria belonging to the species *Chlamydia pneumoniae* characterized in that it comprises the following components:
a) a nucleotide primer or probe capable of hybridizing with a polynucleotide according to the invention.

The disclosure also relates to a kit or set for the detection and/or the identification of bacteria belonging to the species *Chlamydia pneumoniae* or to an associated microorganism, characterized in that it comprises the following components:
a) a nucleotide probe, called capture probe,
b) an oligonucleotide probe, called detection probe ;
c) where appropriate, at least one primer according to the invention as well as the reagents (e.g., polymerase and/or deoxynucleotide triphosphates) necessary for a DNA amplification reaction.

The disclosure so relates to a kit or set for the detection and/or the identification of bacteria belonging to the species *Chlamydia pneumoniae* or to an associated microorganism, characterized in that it comprises the following components:
a) at least one primer ;
b) where appropriate, the reagents necessary for carrying out a DNA amplification reaction;
c) where appropriate, a component which makes,it possible to check the sequence of the amplified fragment, more particularly an oligonucleotide probe according to the invention.

The disclosure relates, in addition, to a kit or set for the detection and/or the identification of bacteria belonging to the species *Chlamydia pneumoniae* or to an associated microorganism, or for the detection and/or the identification of a microorganism characterized in that it comprises a DNA chip according to the invention.

The invention also relates to a method or to a kit or set according to the invention for the detection and/or the identification of bacteria belonging to the species *Chlamydia pneumoniae,* comprising a polypeptide according to the invention and an antibody according to the invention.

According to the present disclosure the said method or the said kit or set above according to the invention, for the detection and/or the identification of bacteria belonging to the species *Chlamydia pneumoniae* is characterized in that the said primer and/or the said probe or the said polypeptides are chosen from the nucleotide sequences or polypeptides according to the invention which have been identified as being specific to the species *Chlamydia pneumoniae* and in that the said antibodies according to the invention are chosen from the antibodies directed against the polypeptides according to the invention chosen from the polypeptides identified as being specific to the species *Chlamydia pneumoniae.*

The invention also comprises screening assays that comprise the steps of:
a) bringing the said compound into contact with an isolated or polynucleotide polypeptide according to the invention ; and
b) determining the capacity of the said compound to bind with the said polypeptide or the said polynucleotide sequence.

The present disclosure is directed to transformed cells according to the invention which may advantageously serve as a model and may be used in methods for studying, identifying and/or selecting compounds capable of being responsible- for pathologies induced or worsened by *Chlamydia pneumoniae,* or capable of preventing and/or of treating these pathologies such as, for example, cardiovascular or respiratory diseases. In particular, the transformed host cells, in particular bacteria of the *Chlamydia* family whose transformation with a vector according to the invention may, for example, increase or inhibit its infectivity, or modulate the pathologies usually induced or worsened by the infection, may be used to infect animals in which the onset of pathologies will be monitored. These nontransformed animals, infected for example with transformed *Chlamydia* bacteria, may serve as a study model.

The compounds capable of being selected may be organic compounds such as polypeptides or carbohydrates or any other organic or inorganic compounds already known, or new organic compounds produced using molecular modeling techniques and obtained by chemical or biochemical synthesis, these techniques being known to persons skilled in the art.

The said selected compounds may be used to modulate the growth and/or the cellular replication of *Chlamydia pneumoniae* or any other associated microorganism and thus to control infection by these microorganisms. The said compounds may also be used to modulate the growth and/or the cellular replication of all eukaryotic or prokaryotic cells, in particular tumour cells and infectious microorganisms, for which the said compounds will prove active, the methods which make it possible to determine the said modulations being well known to persons skilled in the art.

The invention also relates to a pharmaceutical composition comprising a polypeptide according to the invention, or an antibody according to the invention; and a pharmaceutically acceptable vehicle.

The present disclosure also relates to a pharmaceutical composition comprising one or more polypeptides according to the invention and/or one or more fusion polypeptides according to the invention. Such compositions further comprise a pharmaceutically acceptable carrier or vehicle. Pharmaceutical compositions include compositions that comprise a polypeptide or fusion polypeptide that immunoreacts with seropositive serum of an individual infected with *Chlamydia pneumoniae.* In one embodiment, a pharmaceutical composition according to the invention or which comprises a polynucleotide, a vector or a cost cell according to the present invention, can be utilized for the prevention or the treatment of an infection by a bacterium belonging to the species *Chlamydia pneumonia*.

The invention relates, in addition, to an immunogenic composition or a vaccine composition, characterized in that it comprises one or more polypeptides according to the invention and/or one or more hybrid (fusion) polypeptides according to the invention. Such compositions further comprise a pharmaceutically acceptable carrier or vehicle. Immunogenic compositions or fusion polypeptide include compositions that comprise a polypeptide that immunoreacts with seropositive serum of an individual infected with *Chlamydia pneumoniae.*

Immunogenic or vaccine compositions can also comprise DNA immunogenic or vaccine compositions comprising polynucleotide sequences of the invention operatively associated with a regulatory sequence that controls gene expression. Such compositions can include compositions that direct expression of a neutralizing epitope of *Chlamydia pneumoniae.*

The present disclosure also relates to the use of a transformed cell according to the invention, for the preparation of a vaccine composition.

The invention also relates to a vaccine composition, characterized in that it contains a nucleotide sequence according to the invention, a vector according to the invention and/or a transformed cell according to the invention.

The invention also relates to the vaccine compositions according to the invention, for the prevention or the treatment of an infection by a bacterium belonging to the species *Chlamydia pneumoniae* or by an associated microorganism.

The present disclosure also relates to the use of DNA encoding polypeptides of *Chlamydia pneumoniae,* in particular antigenic determinants, to be formulated as vaccine compositions. In accordance with this aspect of the invention, the DNA of interest is engineered into an expression vector under the control of regulatory elements, which will promote expression of the DNA, *i.e.*, promoter or enhancer elements. The promoter element may be cell-specific and permit substantial transcription of the DNA only in predetermined cells. The DNA may be introduced directly into the host either as naked DNA or formulated in compositions with other agents which may facilitate uptake of the DNA including viral vectors, *i.e*., adenovirus vectors, or agents which facilitate immunization, such as bupivicaine and other local anesthetics, saponins and cationic polyamines.

The DNA sequence encoding the antigenic polypeptide and regulatory element may be inserted into a stable cell line or cloned microorganism, using techniques, such as targeted homologous recombination, which are well known to those of skill in the art, and described *e.g*., in Chappel, U.S. Patent No. 4,215,051; Skoultchi, WO 91/06667.

Such cell lines and microorganisms may be formulated for vaccine purposes. The DNA sequence encoding the antigenic polypeptide and regulatory element may be delivered to a mammalian host and introduced into the host genome via homologous recombination.

It is disclosed that, the immunogenic and/or vaccine compositions according to the invention which are intended for the prevention and/or the treatment of an infection by *Chlamydia pneumoniae* will be chosen from the immunogenic and/or vaccine compositions comprising a polypeptide or one of its representative fragments corresponding to a protein, or one of its representative fragments, of the cellular envelope of *Chlamydia pneumoniae.* The vaccine compositions comprising nucleotide sequences will also comprise nucleotide sequences encoding a polypeptide or one of its representative fragments corresponding to a protein, or one of its representative fragments, of the cellular envelope of *Chlamydia pneumoniae.*

The polypeptides or their representative fragments entering into the immunogenic compositions according to the invention may be selected by techniques known to persons skilled in the art, such as for example on the capacity of the said polypeptides to stimulate T cells, which results, for example, in their proliferation or the section of interleukins, and which leads to the production of antibodies directed against the said polypeptides.

In mice, in which a weight dose of the vaccine composition comparable to the dose used in humans is administered, the antibody reaction is tested by collecting serum followed by a study of the formation of a complex between the antibodies present in the serum and the antigen of the vaccine composition, according to the customary techniques.

It is disclosed that vaccine compositions will be preferably in combination with a pharmaceutically acceptable vehicle and, where appropriate, with one or more appropriate immunity adjuvants.

Various types of vaccines are currently available for protecting humans against infectious diseases: attenuated live microorganisms (*M. bovis* - BCG for tubemulosis), inactivated microorganisms (influenza virus), acellular extracts (*Bordetella pertussis* for whooping cough). recombinant proteins (hepatitis B virus surface antigen), polysaccharides (pneumococci). Experiments are underway on vaccines prepared from synthetic peptides or from genetically modified microorganisms expressing heterologous antigens. Even more recently, recombinant plasmid DNAs carrying genes encoding protective antigens were proposed as an alternative vaccine strategy. This type of vaccination is carried out with a particular plasmid derived from an *E*. *coli* plasmid which does not replicate *in vivo* and which encodes only the vaccinal protein. Animals were immunized by simply injecting the naked plasmid DNA into the muscle. This technique leads to the expression of the vaccine protein *in situ* and to a cell-type (CTL) and a humoral type (antibody) immune response. This double induction of the immune response is one of the main advantages of the technique of vaccination with naked DNA.

The vaccine compositions can be evaluated in *in vitro* and *in vivo* animal models prior to host, e.g., human, administration. For example, *in vitro* neutralization assays such as those described by Peterson et al. (1988) can be utilized. The assay described by Peterson et al. (1988) is suitable for testing vaccine compositions directed toward either *Chlamydia pneumoniae* or *Chlamydia trachomatis.*

Briefly, hyper-immune antisera is diluted in PBS containing 5% guinea pig serum, as a complement source. *Chlamydiae* (10⁴ IFU; infectious units) are added to the antisera dilutions. The antigen-antibody mixtures are incubated at 37EC for 45 minutes and inoculated into duplicate confluent Hep-2 or HeLa cell monolayers contained in glass vials (e.g., 15 by 45 mm), which have been washed twice with PBS prior to inoculation. The monolayer cells are infected by centrifugation at 1000X g for 1 hour followed by stationary incubation at 37E for 1 hour. Infected monolayers are incubated for 48 or 72 hours, fixed and stained with a *Chlamydiae* specific antibody, such as anti-MOMP for *C.trachomatis, etc.* IFUs are counted in ten fields at a magnification of 200X. Neutralization titer is assigned based on the dilution that gives 50% inhibition as compared to control monolayers/IFU.

The efficacy of vaccine compositions can be determined *in vivo* by challenging animal models of *Chlamydia pneumoniae* infection, e.g., mice or rabbits, with the vaccine compositions. For example, *in vivo* vaccine composition challenge studies can be performed in the murine model of *Chlamydia pneumonia* infection described by Moazed et al. (1997). Briefly, male homozygous apoE deficient and/or C57 BL/6J mice are immunized with vaccine compositions. Post-vaccination, the mice are mildly sedated by subcutaneous injection of a mixture of ketamine and xylazine, and inoculated intranasally with a total volume of 0.03-0.05 ml of organisms suspended in SPG medium or with SPG alone. The inoculations of *Chlamydia pneumoniae* are approximately 3x10⁷ IFU/mouse. The mice are inoculated with *Chlamydia pneumoniae* at 8, 10, and 12 weeks of age. Tissues are then collected from the lung, spleen, heart, etc. at 1-20 weeks after the first inoculation. The presence of organisms is scored using PCR, histology and immunocytochemistry, or by quantitative culture/IFU after tissue homogenization.

Alternatively, *in vivo* vaccine composition challenge studies can be performed in the rabbit model of *Chlamydia pneumonia* described by Laitinen et al. (1997). Briefly, New Zealand white rabbits (5 months old) are immunized with the vaccine compositions. Post-vaccination, the rabbits are sedated with Hypnorm, 0.3 ml/Kg of body weight, intramuscularly, and inoculated intranasally with a total of 0.5 ml of *Chlamydia pneumoniae* suspended in SPG medium or with SPG alone. The inoculations of *Chlamydia pneumoniae* are approximately 3x10⁷ IFU/rabbit. The rabbits are reinfected in the same manner and with the same dose 3 weeks after the primary inoculation. Tissues are then collected 2 weeks after the primary infection and 1, 2, and 4 weeks after the reinjection. The presence of *Chlamydia pneumoniae* is scored using PCR, histology and immunocytochemistry, or by quantitative culture/IFU after tissue homogenization.

The vaccine compositions comprising nucleotide sequences or vectors into which the said sequences are inserted are in particular described in International Application No. WO 90/11092 and also in International Application No. WO 95/11307.

It is disclosed that the nucleotide sequence constituting the vaccine composition according to the invention may be injected into the host after having been coupled to compounds which promote the penetration of this polynucleotide inside the cell or its transport up to the cell nucleus. The resulting conjugates may be encapsulated into polymeric microparticles, as described in International Application No. WO 94/27238 (Medisorb Technologies International).

It is disclosed that the nucleotide sequence, preferably a DNA, of the vaccin composition is complexed with the DEAE-dextran (Pagano et al., 1967) or with nuclear proteins (Kaneda et al., 1989), with lipids (Felgner et al., 1987) or encapsulated into liposomes (Fraley et al., 1980) or alternatively introduced in the form of a gel facilitating its transfection into the cells (Midoux et al., 1993, Pastore et al., 1994). The polynucleotide or the vector according to the invention may also be in suspension in a buffer solution or may be combined with liposomes.

Advantageously, such a vaccine will be prepared in accordance with the technique described by Tacson et al. or Huygen et al. in 1996 or alternatively in accordance with the technique described by Davis et al. in International Application No. WO 95/11307.

Such a vaccine may also be prepared in the form of a composition containing a vector according to the invention, placed under the control of regulatory elements allowing its expression in humans or animals. It is possible, for example, to use, as vector for the *in vivo* expression of the polypeptide antigen of interest, the plasmid pcDNA3 or the plasmid pcDNAl/neo, both marketed by Invitrogen ® & D Systems, Abingdon, United Kingdom). It is also possible to use the plasmid VIIns.tPA, described by Shiver et al. in 1995. Such a vaccine will advantageously comprise, in addition to the recombinant vector a saline solution for example a sodium chloride solution.

It is also disclosed that the immunogenic compositions of the invention can also be utilized as part of methods for immunization, wherein such methods comprise administering to a host, e.g., a human host, an immunizing amount of the immunogenic compositions of the invention. The method of immunizing is a method of immunizing against *Chlamydia pneumoniae.*

A pharmaceutically acceptable vehicle is understood to designate a compound or a combination of compounds entering into a pharmaceutical or vaccine composition which does not cause side effects and which makes it possible, for example, to facilitate the administration of the active compound, to increase its life and/or its efficacy in the body, to increase its solubility in solution or alternatively to enhance its preservation. These pharmaceutically acceptable vehicles are well known and will be adapted by persons skilled in the art according to the nature and the mode of administration of the active compound chosen.

As regards the vaccine formulations, these may comprise appropriate immunity adjuvants which are known to persons skilled in the art, such as, for example, aluminum hydroxide, a representative of the family of muramyl peptides such as one of the peptide derivatives of N-acetyl-muramyl, a bacterial lysate, or alternatively incomplete Freund's adjuvant, Stimulon™ QS-21 (Aquila Biopharmaceuticals, Inc., Framingham, MA), MPL™ (3-O-deacylated monophosphoryl lipid A; RIBI ImmunoChem Research, Inc., Hamilton, MT), aluminum phosphate, IL-12 (Genetics Institute, Cambridge, MA).

These compounds will be administered by the systemic route, in particular by the intravenous route, by the intranasal, intramuscular, intradermal or subcutaneous route, or by the oral route. The vaccine composition comprising polypeptides according to the invention will be administered several times, spread out over time, by the intradermal or subcutaneous route.

Their optimum modes of administration, dosages and galenic forms may be determined according to criteria which are generally taken into account in establishing a treatment adapted to a patient, such as for example the patient's age or body weight, the seriousness of his general condition, tolerance of the treatment and the side effects observed.

### Legend to the figures :

Figure 1 : Line for the production of *Chlamydia pneumoniae* sequences Figure 2 : Analysis of the sequences and assembling
Figure 3 : Finishing techniques
   Figure 3a) : Assembly map
   Figure 3b) : Determination and use of the orphan ends of the contigs

### EXAMPLES

### Experimental procedures

### Cells

The *Chlamydia pneumoniae* strain (CM1) used by the inventors is obtained from ATCC (American Culture Type Collection) where it has the reference number ATCC 1360-VR.

It is cultured on HeLa 229 cells, obtained from the American Type Culture Collection, under the reference ATCC CCL-2.1.

### Culture of the cells

The HeLa ATCC CCL-2.1 cells are cultured in 75-ml cell culture flasks (Coming). The culture medium is Dulbecco's modified cell culture medium (Gibco BRL No. 04101965) supplemented with MEM amino acids (Gibco BRL - No. 04301140) L (5 ml per 500 ml of medium) and 5% foetal calf serum (Gibco BRL No. 10270 batch 40G8260K) without antibiotics or antifungals.

The cell culture stock is maintained in the following manner. The cell cultures are examined under an inverted microscope. 24 hours after confluence, each cellular lawn is washed with PBS (Gibco BRL No. 04114190), rinsed and then placed for 5 min in an oven in the presence of 3 ml of trypsine (Gibco BRL No. 25200056). The cellular lawn is then detached and then resuspended in 120 ml of culture medium, the whole is stirred in order to make the cellular suspension homogeneous. 30 ml of this suspension are then distributed per cell culture flask. The flasks are kept in a CO₂ oven (5%) for 48 hours at a temperature of 37°C. The cell stock is maintained so as to have available daily 16 flasks of subconfluent cells. It is these subconfluent cells which will be used so as to be infected with Chlamydia. 25-ml cell culture flasks are also used, these flasks are prepared in a similar manner but the volumes used for maintaining the cells are the following: 1 ml of trypsine, 28 ml of culture medium to resuspend the cells, 7 ml of culture medium are used per 25-ml flask.

### Infection of the cells with Chlamydia

Initially, the Chlamydiae are obtained frozen from ATCC (-70°C), in suspension in a volume of 1 ml. This preparation is slowly thawed, 500 µl are collected and brought into contact with subconfluent cells, which are obtained as indicated above, in a 25-ml cell culture flask, containing 1 ml of medium, so as to cover the cells. The flask is then centrifuged at 2000 rpm in a "swing" rotor for microtitre plates, the centrifuge being maintained at a temperature of 35°C. After centrifugation, the two flasks are placed in an oven at 35°C for three hours. 6 ml of culture medium containing cycloheximide (1 µg/ml) are then added and the flask is stored at 35°C. After 72 hours, the level of infection is evaluated by direct immunofluorescence and by the cytopathogenic effect caused to the cells.

### Direct immunofluorescence

Starting with infected cells, which were obtained as indicated above, a cellular smear is deposited with a Pasteur pipette on a microscope slide. The cellular smear is fixed with acetone for 10 minutes; after draining the acetone, the smear is covered with 30 µl of murine monoclonal antibodies directed against MOMP (major outer membrane protein) of Chlamydia (Syva, Biomérieux) labelled with fluorescein isothiocyanate. The whole is then incubated in a humid chamber at a temperature of 37°C. The slides are then rinsed with water, slightly dried, and then after depositing a drop of mounting medium, a coverslip is mounted before reading. The reading is carried out with the aid of a fluorescence microscope equipped with the required filters (excitation at 490 nm, emission at 520 nm).

### Harvesting of the Chlamvdia pneumoniae

After checking the infection by direct immunofluorescence, carried out as indicated above, the culture flasks are opened under a sterile cabinet, sterile glass beads with a diameter of the order of a millimeter are placed in the flask. The flask is closed and then vigorously stirred while being maintained horizontally, the cellular lawn at the bottom, so that the glass beads can have a mechanical action on the cellular lawn. Most of the cells are thus detached or broken; the effect of the stirring is observed under an optical microscope so as to ensure proper release of Chlamydiae.

### Large-scale infection of the cell cultures

The product of the Chlamydiae harvest (culture medium and cellular debris) is collected with a pipette, and distributed into three cell culture flasks containing subconfluent HeLa ATCC CCL-2.1 cells, obtained as indicated above. The cells thus inoculated are placed under gentle stirring (swing) in an oven at 35°C. After one hour, the flasks are kept horizontally in an oven so that the culture medium covers the cells for 3 hours. 30 ml of culture medium containing actydione (1 µg/ml) are then added to each of the flasks. The culture flasks are then stored at 35°C for 72 hours. The cells thus infected are examined under an optical microscope after 24 hours, the cytopathogenic effect is evaluated by the appearance of cytoplasmic inclusions which are visible under an inverted optical microscope. After 72 hours, the vacuoles containing the Chlamydiae occupy the cytoplasm of the cell and push the cell nucleus sideways. At this stage, numerous cells are spontaneously destroyed and have left free elementary bodies in the culture medium. The Chlamydiae are harvested as described above and are either frozen at -80°C or used for another propagation.

### Purification of the Chlamydiae

The product of the Chlamydia harvests is stored at -80°C and thawed on a water bath at room temperature. After thawing, each tube is vigorously stirred for one minute and immersed for one minute in an ultrasound tank (BRANSON 1200); the tubes are then stirred by inverting before being centrifuged for 5 min at 2000 rpm. The supernatant is carefully removed and kept at cold temperature (ice). The supernatant is vigorously stirred and then filtered on nylon filters having pores of 5 microns in diameter on a support (Nalgene) allowing a delicate vacuum to be established under the nylon filter. For each filtration, three nylon filters are superposed; these filters are replaced after every 40 ml of filtrate. Two hundred milliliters of filtration product are kept at cold temperature, and then after stirring by inverting, are centrifuged at 10,000 rpm for 90 min, the supernatant is removed and the pellet is taken up in 10 ml of 10 mM Tris, vigorously vortexed and then centrifuged at 10,000 rpm for 90 min. The supernatant is removed and the pellet is taken up in a buffer (20 mM Tris pH 8.0, 50 mM KCI, 5 mM MgCl₂) to which 800 units of DNAse I (Boehringer) are added. The whole is kept at 37°C for one hour. One ml of 0.5 M EDTA is then added, the whole is vortexed and frozen at -20°C.

### Preparation of the DNA

The Chlamydiae purified above are thawed and subjected to a proteinase K (Boehringer) digestion in a final volume of 10 ml. The digestion conditions are the following: 0.1 mg/ml proteinase K, 0.1 × SDS at 55EC, stirring every 10 min. The product of digestion is then subjected to a double extraction with phenol-chloroform, two volumes of ethanol are added and the DNA is directly recovered with a Pasteur pipette having one end in the form of a hook. The DNA is dried on the edge of the tube and then resuspended in 500 µl of 2 mM Tris pH 7.5. The DNA is stored at 4°C for at least 24 hours before being used for the cloning.

### Cloning of the DNA

After precipitation, the DNA is quantified by measuring the optical density at 260 nm. Thirty µg of Chlamydia DNA are distributed into 10 tubes of 1.5 ml and diluted in 300 µl of water. Each of the tubes is subjected to 10 applications of ultrasound lasting for 0.5 sec in a sonicator (unisonix XL2020). The contents of the 10 tubes are then grouped and concentrated by successive extractions with butanol (Sigma B1888) in the following manner: two volumes of butanol are added to the dilute DNA mixture. After stirring, the whole is centrifuged for five minutes at 2500 rpm and the butanol is removed. This operation is repeated until the volume of the aqueous phase is less than 1 ml. The DNA is then precipitated in the presence of ethanol and of 0.5 M sodium acetate pH 5.4, and then centrifuged for thirty minutes at 15,000 rpm at cold temperature (4°C). The pellet is washed with 75% ethanol, centrifuged for five minutes at 15,000 rpm and dried at room temperature. A tenth of the preparation is analysed on a 0.8% agarose gel. Typically, the size of the DNA fragments thus prepared is between 200 and 8000 base pairs.

To allow the cloning of the DNA obtained, the ends are repaired. The DNA is distributed in an amount of 10 µg/tube, in the following reaction medium: 100 µl final volume, 1 × buffer (Biolabs 201L), 0.5 µl BSA 0.05 mg/ml, 0.1 mM dATP, 0.1 mM each of dGTP, dCTP or dTTP, 60,000 IU T4 DNA polymerase. The reaction is incubated for thirty minutes at 16°C. The contents of each of the tubes are then grouped before carrying out an extraction with phenol-chloroform and then precipitating the aqueous phase as described above. After this step, the DNA thus prepared is phosphorylated. For that, the DNA is distributed into tubes in an amount of 10 µg per tube, and then in a final volume of 50 µl, the reaction is prepared in the following manner: 1 mM ATP, 1 × kinase buffer, 10 IU T4 polynucleotide kinase (Biolabs 201L). The preparation is incubated for thirty minutes at 37°C. The contents of the tubes are combined and a phenol-chloroform extraction and then a precipitation are carried out in order to precipitate the DNA. The latter is then suspended in 1 µl of water and then the DNA fragments are separated according to their size on a 0.8% agarose gel (1 × TAE). The DNA is subjected to an electric field of 5 V/cm and then visualized on a UV table. The fragments whose size varies between 1200 and 2000 base pairs are selected by cutting out the gel. The gel fragment thus isolated is placed in a tube and then the DNA is purified with the Qiaex kit (20021 Qiagen), according to the procedure provided by the manufacturer.

### Preparation of the vector

14 µg of the cloning vector pGEM-5Zf (Proméga P2241) are diluted in a final volume of 150µl and are subjected to digestion with the restriction enzyme EcoRV 300 IU (Biolabs 195S) according to the protocol and with the reagents provided by the manufacturer. The whole is placed at 37°C for 150 min and then distributed in the wells of a 0.8% agarose gel subjected to an electric field of 5 V/cm. The linearized vector is visualized on a UV table, isolated by cutting out the gel and then purified by the Qiaex kit (Qiagen 20021) according to the manufacturer's recommendations. The purification products are grouped in a tube, the volume is measured and then half the volume of phenol is added and the whole is vigorously stirred for 1 min. Half the volume of chloroform-isoamyl alcohol 24:1 is added and vigorously stirred for 1 min. The whole is centrifuged at 15,000 rpm for 5 min at 4°C, the aqueous phase is recovered and transferred into a tube. The DNA is precipitated in the presence of 0.3 M sodium acetate, pH 5.4 and 3 volumes of ethanol and placed at -20°C for I hour. The DNA is then centrifuged at 15,000 rpm for 30 min at 4°C, the supernatant is removed while preserving the pellet, washed twice with 70% ethanol. After drying at room temperature, the DNA is suspended in 25 µl of water.

### Phosphorylation of the vector

25 µl of the vector prepared in the preceding step are diluted in a final volume of 500 µl of the following reaction mixture:

After repair, the DNA is subjected to a phenol-chloroform extraction and a precipitation, the pellet is then taken up in 10 µl of water, the DNA is quantified by measuring the optical density at 260 nm. The quantified DNA is ligated into the vector PGEm-5Zf(+) prepared by the restriction enzyme EcoRV and dephosphorylated (see preparation of the vector). The ligation is carried out under three conditions which vary in the ratio between the number of vector molecules and the number of insert molecules. Typically, an equimolar ratio, a ratio of 1:3 and a ratio of 3:1 are used for the ligations which are, moreover, carried out under the following conditions: vector PGEm-5Zf(+) 25 ng, cut DNA, ligation buffer in a final volume of 20 µl with T4 DNA ligase (Amersham E70042X); the whole is then placed in a refrigerator overnight and then a phenol-chloroform extraction and a precipitation are carried out in a conventional manner. The pellet is taken up in 5 µl of water.

### Transformation of the bacteria

### Plating of the bacteria

Petri dishes containing LB Agar medium containing ampicillin (50 µg/ml), Xgal (280 µg/ml) [5-bromo-4-chloro-indolyl-beta-D-galactopyranoside (Sigma B-4252)], IPTG (140 µg/lg/ml) [isopropyl-beta-D-thiogalactoside (Sigma I-6758)] are used, 50 and 100 µl of bacteria are plated for each of the ligations. The Petri dishes are placed upside down at 37°C for 15 to 16 hours in an oven. The number of "recombinant" positive clones is evaluated by counting the white colonies and the blue colonies which are thought to contain the vector alone.

### Evaluation of the "recombinant" positive clones

Ninety-four white colonies and two blue colonies are collected with the aid of sterile cones and are deposited at the bottom of the wells of plates designed for carrying out the amplification techniques. 30 µl of the following reaction mixture are added to each well: 1.7 mM MgCl₂, 0.2 mM each of dATP, dCTP, dGTP and dTTP, two synthetic oligonucleotides corresponding to sequences flanking the cloning site on either side and orienting the synthesis of the DNA in a convergent manner (0.5 µM RP and PU primers, 1 U TAQ polymerase (GibcoBRL 18038-026)).

The colonies thus prepared are subjected to a temperature of 94°C for 5 min and then to 30 thermal cycles composed of the following steps: 94°C for 40 s, 50°C for 30 s, 72°C for 180 s. The reaction is then kept for 7 min at 72°C and then kept at 4°C.

The amplification products are deposited on an agarose gel (0.8%), stained with ethidium bromide, subjected to electrophoresis, and then analysed on an ultraviolet table. The presence of an amplification fragment having a size greater than 500 base pairs indicates the presence of an insert. The bacterial clones are then prepared so as to study the sequence of their insert.

### Sequencing

To sequence the inserts of the clones obtained as above, these were amplified by PCR on bacteria cultures carried out overnight using the primers for the vectors flanking the inserts. The sequence of the ends of these inserts (on average 500 bases on each side) was determined by automated fluorescent sequencing on an ABI 377 sequencer, equipped with the ABI Prism DNA Sequencing Analysis software (version 2.1.2).

### Analysis of the sequences

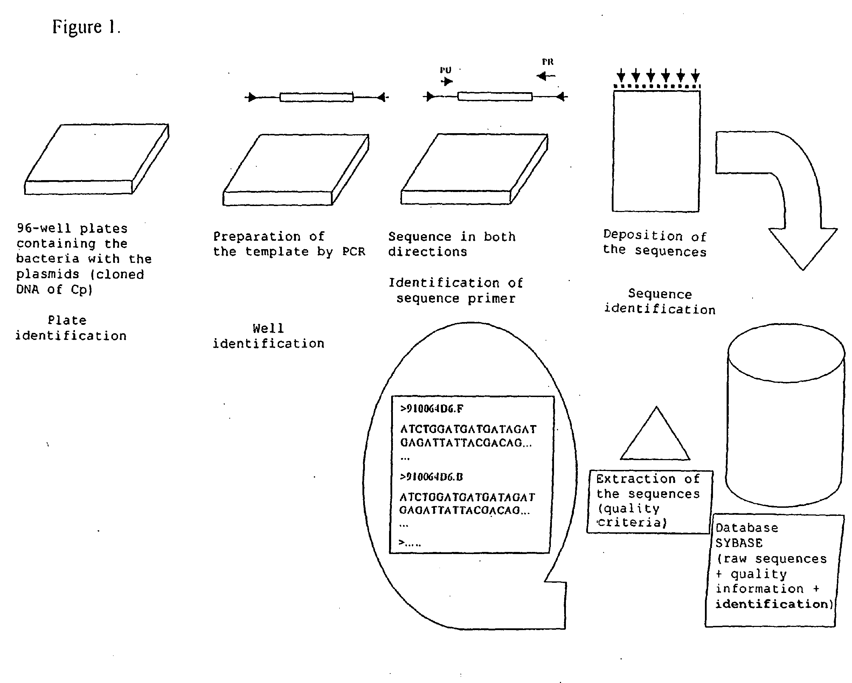

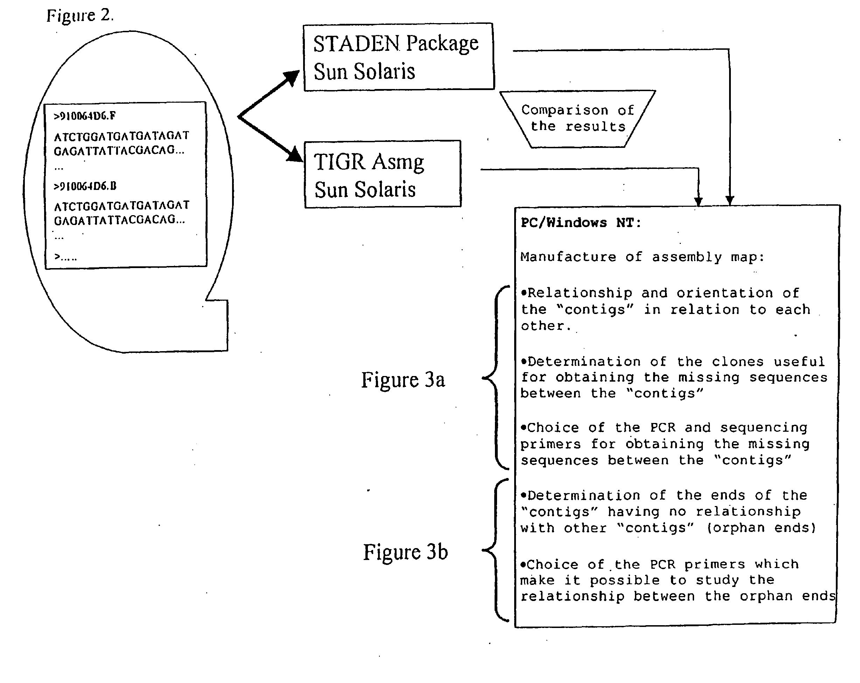

The sequences obtained by sequencing in a high-yield line (Figure 1) are stored in a database; this part of the production is independent of any treatment of the sequences. The sequences are extracted from the database, avoiding all the regions of inadequate quality, that is to say the regions for which uncertainties are observed on the sequence at more than 95%. After extraction, the sequences are introduced into a processing line, the diagram of which is described in Figure 2. In a first path of this processing line, the sequences are assembled by the Gap4 software from R. Staden (Bonfield et al., 1995) (OS UNIX/SUN Solaris); the results obtained by this software are kept in the form of two files which will be used for a subsequent processing. The first of these files provides information on the sequence of each of the contigs obtained. The second file represents all the clones participating in the composition of all the contigs as well as their positions on the respective contigs.

The second processing path uses a sequence assembler (TIGR-Asmg assembler UNIX/SUN Solaris); the results of this second processing path are kept in the form of a file in the TIGR-Asmg format which provides information on the relationship existing between the sequences selected for the assembly. This assembler is sometimes incapable of linking contigs whose ends overlap over several hundreds of base pairs.

The results obtained from these two assemblers are compared with the aid of the BLAST program, each of the contigs derived from one assembly path being compared with the contigs derived from the other path.

For the two processing paths, the strict assembly parameters are fixed (95% homology, 30 superposition nucleotides). These parameters avoid 3 to 5% of the clones derived from eukaryotic cells being confused with sequences obtained from the clones derived from *Chlamydia pneumoniae.* The eukaryotic sequences are however preserved during the course of this project; the strategy introduced, which is described below, will be designed, inter alia, not to be impeded by these sequences derived from contaminating clones.

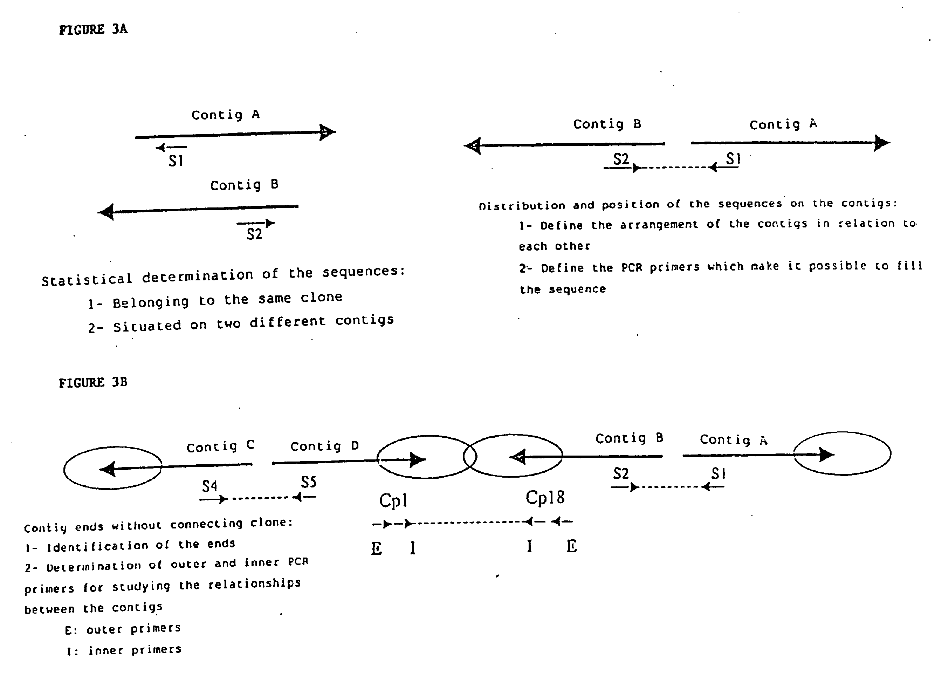

The results of these two assemblers are processed in a software developed for this project. This software operates on a Windows NT platform and receives, as data, the results derived from the STADEN software and/or the results derived from the TIGR-Asmg assembler, the software, results, after processing of the data, in the determination of an assembly map which gives the proximity relationship and the orientation of the contigs in relation to one another (Figure 3a). Using this assembly map, the software determines all the primers necessary for finishing the project. This treatment, which will be detailed below, has the advantage of distinguishing the isolated sequences derived from the contaminations, by the DNA eukaryotic cells, of the small-sized sequences clearly integrated into the project by the relationships which they establish with contigs. In order to allow, without any risk of error, the arrangement and the orientation of the contigs in relation to one another, a statistical evaluation of the accuracy of the names (naming) "naming" of sequence is made from the results of "contigation". This evaluation makes it possible to give each of the clone plates, as well as each of the subsets of plates, a weight which is inversely proportional to probable error rate existing in the "naming" of the sequences obtained from this plate or from a subset of this plate. In spite of a low error rate, errors may occur throughout the steps of production of the clones and of the sequences. These steps are numerous, repetitive and although most of them are automated, others, like the deposition in the sequencers, are manual; it is then possible for the operator to make mistakes such as the inversion of two sequences. This type of error has a repercussion on the subsequent processing of the data, by resulting in relationships (between the contigs) which do not exist in reality, then in attempts at directed sequencing between the contigs which will end in failure. It is because of this that the evaluation of the naming errors is of particular importance since it allows the establishment of a probabilistic assembly map from which it becomes possible to determine all the clones which will serve as template to obtain sequences separating two adjacent contigs. Table 2 of parent U.S. application serial No. 60/107078 filed November 4, 1998 and French application 97-14673 filed November 21, 1997, each of which is incorporated by reference herein in its entirety, gives the clones and the sequences of the primers initially used during the initial operations.

To avoid the step which consists in ordering and then preparing the clones by conventional microbiological means, outer and inner primers oriented towards the regions not yet sequenced are defined by the software. The primers thus determined make it possible to prepare, by PCR, a template covering the nonsequenced region. It is the so-called outer primers (the ones most distant from the region to be sequenced) which are used to prepare this template. The template is then purified and a sequence is obtained on each of the two strands during 2 sequencing reactions which each use one of the 2 inner primers. In order to facilitate the use of this approach, the two outer primers and the two inner primers are prepared and then stored on the same position of 4 different 96-well plates. The two plates containing the outer primers are used to perform the PCRs which will serve to prepare the templates. These templates will be purified on purification columns preserving the topography of the plates. Each of the sequences will be obtained using primers situated on one and then on the other of the plates containing the inner primers. This distribution allows a very extensive automation of the process and results in a method which is simple to use for finishing the regions not yet sequenced. Table 3 of parent U.S. application serial No. 60/107078 filed November 4, 1998 and French application 97-14673 filed November 21, 1997, each of which is incorporated by reference herein in its entirety, gives the names and the sequences of the primers used for finishing *Chlamydia pneumoniae.*

Finally, a number of contigs exist in a configuration where one of their ends is not linked to any other contig end (Figure 3b) by a connecting clone relationship (a connecting clone is defined as a clone having one sequence end on a contig and the other end of its sequence on another contig; furthermore, this clone must be derived from a plate or a subset of plates with adequate naming quality). For the *Chlamydia pneumoniae* project, this particular case occurred 24 times. Two adjacent PCR primers orienting the synthesis of the DNA towards the end of the consensus sequence are defined for each of the orphan ends of the consensus sequence. The primer which is closest to the end of the sequence is called the inner primer whereas the primer which is more distant from the end of the sequence is called the outer primer. The outer primers are used to explore the mutual relationship between the orphan ends of the different contigs. The presence of a single PCR product and the possibility of amplifying this product unambiguously using the inner primers evokes the probable relationship between the contigs on which the primers which allowed the amplification are situated. This relationship will be confirmed by sequencing and will allow the connection between the orphan ends of the consensus sequences. This strategy has made it possible to obtain a complete map of the *Chlamydia pneumoniae* chromosome and then to finish the project.

### Quality control

All the bases not determined with certainty in the chromosomal sequence were noted and the density of uncertainties was measured on the entire chromosome. The regions with a high density of uncertainties were noted and the PCR primers spanning these regions were drawn and are represented in Table 4 of parent U.S. application serial No. 60/107078 filed November 4, 1998 and French application 97-14673 filed November 21, 1997 each of which is incorporated by reference herein in its entirety.

The sequence of each of the PCR products was obtained with two operational primers different from the amplification primers. The sequences were obtained in both directions for all the PCRs (100% success).

### Data banks

Local reorganizations of major public banks were used. The protein bank used consists of the nonredundant fusion of the Genpept bank (automated translation of GenBank, NCBI; Benson et al., 1996).

The entire BLAST software (public domain, Altschul et al., 1990) for searching for homologies between a sequence and protein or nucleic data banks was used. The significance levels used depend on the length and the complexity of the region tested as well as the size of the reference bank. They were adjusted and adapted to each analysis.

The results of the search for homologies between a sequence according to the invention and protein or nucleic data banks are presented and summarized in Table 1 below.

Table 1: List of coding chromosome regions and homologies between these regions and the sequence banks.

Legend to Table 1: Open reading frames are identified with the GenMark software version 23A (GenePro), the template used is *Chlamydia pneumoniae* of order 4 on a length of 196 nucleotides with a window of 12 nucleotides and a minimum signal of 0.5. The reading frame ORF 291 to ORF is numbered in order of appearance on the chromosome. The positions of the beginning and of the end are then given in column 2 (position). When the position of the beginning is greater than the position of the end, this means that the region is encoded by the strand complementary to the sequence which was given in the sequence SEQ ID No. 1.

All the putative products were subjected to a search for homology on GENPEPT (release 102 for SEQ ID No. 291) with the BLASTP software (Altschul et al. 1990). With, as parameters, the default parameters with the exception of the expected value E set at 10⁻⁵ (for SEQ ID No. 291). Subsequently, only the identities greater than 30% (I% column) were taken into account. The description of the most homologous sequence is given in the Homology column; the identifier for the latter sequence is given in the ID column and the animal species to which this sequence belongs is given in the Species column. The Homology score is evaluated by the sum of the blast scores for each region of homology and reported in the Score column.

### Materials and Methods for transmembrane domains:

The DAS software was used as recommended by the authors (Cserzo et al., 1997).

This method uses, to predict the transmembrane domains, templates derived from a sampling of selected proteins. All the regions for which a "Cutoff" greater than 1.5 was found by the program were taken into account.

### Additional ORF Finder Programs

For this analysis, two additional ORF finder programs were used to predict potential open reading frames of a minimum length of 74 amino acids; Glimmer (Salzberg, S.L., Delcher, A., Kasif, S., and W. White. 1998. Microbial gene identification using interpolated Markov models. Nucleic Acids Res. 26:544-548.), and an in-house written program. The in-house program used a very simple search algorithm. The analysis required the that the genomic DNA sequence text be in the 5' to 3' direction, the genome is circular, and that TAA, TAG, and TGA are stop codons. The search parameters were as follows:
(1) A search for an ORF that started with a GTG codon was performed. If no GTG codons were found, then a search for an ATG codon was performed. However, if a GTG codon was found, then a search downstream for a ATG codon was performed. All start and stop nucleotide positions were recorded.
(2) A search for an ORF that started with a TTG codon was performed. If no TTG codons were found, then a search for a ATG codon was performed. However, if a TTG codon was found, then a search downstream for a ATG codon was performed. All start and stop nucleotide positions were recorded.
(3) The analysis described in steps 1 and 2 were repeated for the opposite strand of DNA sequence.
(4) A search for ORFs that determined all ORF lengths using start and stop positions in the same reading frames was performed.
(5) All ORFs whose DNA length was less than 225 nucleotides were eliminated from the search.

### Surface Exposed Protein Search Criteria

Potential cell surface vaccine targets are outer membrane proteins such as porins, lipoproteins, adhesions and other non-integral proteins. In *Chlamydia psittaci,* the major immunogens is a group of putative outer membrane proteins (POMPs) and no homologs have been found in *Chlamydia pneumoniae* and *Chlamydia trachomatis* by traditional analysis (Longbottom, D., Russell, M., Dunbar, S.M., Jones, G.E., and A.J. Herring. 1998. Molecular Cloning and Characterization of the Genes Coding for the Highly Immunogenic Cluster of 90-Kilodalton Envelope Proteins from Chlamydia psittaci Subtype That Causes Abortion in Sheep. Infect Immun 66:1317-1324.) Several putative outer membrane proteins have been identified in *Chlamydia pneumoniae,* all of which may represent vaccine candidates. The major outer membrane protein (MOMP) gene (omp1) has been found in various isolates of *Chlamydia pneumoniae* (Jantos, CA., Heck, S., Roggendorf, R., Sen-Gupta, M., and Hegemann, JH. 1997. Antigenic and molecular analyses of different chlamydia pneumoniae strains. J. Clin Microbiology 35(3):620-623.) Various criteria, as listed below, were used to identify putative surface exposed ORFs from the genomic DNA sequence of *Chlamydia pneumoniae* (French application 97-14673 filed 21 November 1997). Any ORF which met any one or more of the individual criteria were listed in this category.

Protein homology searches were done using the Blastp 2.0 tool (Altschul, S.F., Madden, T.L., Schaffer, A.A., Zhang, J., Zhang, Z., Miller, W., and D.J. Lipman. 1997. Gapped BLAST and PSI-BLAST: a new generation of protein database search programs. Nucleic Acids Res. 25:3389-3402.) An ORF product was labeled surface exposed if there was homology to a known, or hypothetical, or putative surface exposed protein with a P score better than e-¹⁰.

Most, if not all, proteins that are localized to the membrane of bacteria, via a secretory pathway, contain a signal peptide. A software program, SignalP, analyzes the amino acid sequence of an ORF for such a signal peptide (Nielsen, H., Engelbrecht. J., Brunak, S., and G. von Heijne. 1997. Identification of prokaryotic and eukaryotic signal peptides and prediction of their cleavage sites. Protein Engineering 10:1-6.) The first 60 N-terminal amino acids of each ORF were analyzed by SignalP using the Gram-Negative software database. The output generates four separate values, maximum C, maximum Y, maximum S, and mean S. The S-score, or signal region, is the probability of the position belonging to the signal peptide. The C-score, or cleavage site, is the probability of the position being the first in the mature protein. The Y-score is the geometric average of the C-score and a smoothed derivative of the S-score. A conclusion of either a Yes or No is given next to each score. If all four conclusions are Yes and the C-terminal amino acid is either a phenylalanine (F) or a tyrosine (Y), the ORF product was labelled outer membrane (Struyve, M., Moons, M., and J. Tommassen. 1991. Carboxy-terminal Phenylalanine is Essential for the Correct Assembly of a Bacterial Outer Membrane Protein. J. Mol. Biol. 218:141-148.)

The program called Psort, determines the localization of a protein based on its signal sequence, recognition of transmembrane segments, and analysis of its amino acid composition (Nakai, K., and M. Kanehisa. 1991. Expert system for predicting protein localization sites in gram-negative bacteria. Proteins 11:95-110.) An ORF product is considered to be an outer membrane protein if the output data predicts the protein as outer membrane with a certainty value of 0.5 or better and whose value is at least twice as large as the next predicted localized certainty value.

Finally, ORF products that were not predicted to be outer membrane or surface exposed, based on the above criteria, were further analyzed. The blastp output data for these ORFs were searched using various general and specific keywords, suggestive of known cell surface exposed proteins. An ORF was labeled surface exposed if the keywords matched had a Blastp hit, had a P score better than e⁻¹⁰, and that there was no better data indicating otherwise. The following is a list of the searched keywords:

| Adhesion Omp | Adhesin Outer Surface | Invasin Porin | Invasion Outer Membrane | Extensin | |
|---|---|---|---|---|---|
| Cell Surface | Cell Wall | Pilus | Pilin | Flagellar sheath | BtuB |
| Cir | ChuA | CopB | ExeD | FadL | FecA |
| FepA | FhuA | FmdC | FomA | FrpB | GspD |
| HemR | HgbA | Hgp | HmbR | HmuR | HMW |
| HrcC | Hrp | InvG | LamB | LbpA | LcrQ |
| Lmp1 | MxiD | MOMP | PilE | HpaA | NolW |
| NspA | OpcP | OpnP | Opr | OspA | PhoE |
| PldA | Por | PscC | PulD | PupA | QuiX |
| RafY | ScrY | SepC | ShuA | SomA | SpiA |
| Tbp1 | Yop | YscC | mip | Tol | |

Those ORFs that did not meet the minimum requirement for being an outer membrane protein based on the above search criteria but which were homologous to identified outer membrane ORFs in *Chlamydia trachomatis* were included. The *Chlamydia trachomatis* genome (French patent applications FR97-15041, filed 28 November 1997 and 97-16034 filed 17 December 1997) was analyzed using the above search criteria and a number of outer membrane ORFs were identified. These *Chlamydia trachomatis* ORFs were then tested against the *Chlamydia pneumoniae* genome using Blastp. Any *Chlamydia pneumoniae* ORF with a Blastp P value better than e⁻¹⁰ against a *Chlamydia trachomatis* outer membrane was included in this section, if there was no better data indicating otherwise. A list of ORFs in the *Chlamydia pneumoniae* genome encoding putative surface exposed proteins is set forth above in the specification.

### Identification of Putative Lipoproteins in the Genome of Chlamydia pneumoniae

Lipoproteins are the most abundant post-translationally modified bacterial secretory proteins (Pugsley, A. P.. 1993. The complete general secretory pathway in Gram-negative bacteria. Microbiol. Rev. 57:50-108). The characteristic features of lipoproteins are a thiol-linked diacylglyceride and an amine-linked monoacyl group on the cysteine that becomes the amino-terminal residue after signal peptide cleavage by Signal Peptidase II. (Pugsley, A. P.. 1993. The complete general secretory pathway in Gram-negative bacteria. Microbiol. Rev. 57:50-108). The identification of putative lipoproteins from the genomic sequencing of *Chlamydia pneumoniae* was done by examining the deduced amino acid sequence of identified ORFs for the presence of a signal peptide with a Signal Peptidase II cleavage site analogous to the consensus sequence for prolipoprotein modification and processing reactions (Hayashi, S., and H. C. Wu. 1992. Identification and characterization of lipid-modified proteins in bacteria, p. 261-285. In N. M. Hooper and A. J. Turner (ed.) Lipid modification of proteins: A practical approach. Oxford University Press, New York; Sutcliffe, I. C. and R. R. B. Russell. 1995. Lipoproteins of Gram-positive bacteria. J. Bacteriol. 177:1123-1128.).

*Chlamydia pneumoniae* ORFs were initially screened for the most basic of lipoprotein characteristics, a cysteine in the first 30 amino acids of the deduced protein. ORFs with a standard start codon (ATG, GTG, or TTG) and having one or more of the following characteristics were selected for direct analysis of their first 30 amino acids:
(a) Significant Signal P value (at least two out of the four values are Yes)
(b) PSORT value indicating membrane passage (IM-inner membrane, Peri-periplasm, or OM-outer membrane)
(c) Identification of the word lipoprotein among the ORF blastp data set.
(d) A Blastp value of <e⁻¹⁰ with a putative lipoprotein from *Chlamydia trachomatis* (French applications 97-15041 filed 28 November 1997 and 97-16034 filed 17 December 1997).

The first 30 amino acids of each ORF in this set were analyzed for the characteristics commonly found in lipoprotein signal peptides (Pugsley, A. P.. 1993. The complete general secretory pathway in Gram-negative bacteria. Microbiol. Rev. 57:50-108; Hayashi, S., and H. C. Wu. 1992. Identification and characterization of lipid- modified proteins in bacteria, p. 261-285. In N. M. Hooper and A. J. Turner (ed.) Lipid modification of proteins: A practical approach. Oxford University Press, New York; Sutcliffe, I. C. and R. R. B. Russell. 1995. Lipoproteins of Gram-positive bacteria. J. Bacteriol. 177:1123-1128.) Putative lipoprotein signal peptides were required to have a cysteine between amino acid 10 and 30 and reach a minimum score of three based on the following criteria for lipoprotein signal peptides:
(a) Identification of specific amino acids in specific positions around the cysteine which are part of the consensus Signal Peptidase II cleavage site (Hayashi, S., and H. C. Wu. 1992. Identification and characterization of lipid-modified proteins in bacteria, p. 261-285. In N. M. Hooper and A. J. Turner (ed.) Lipid modification of proteins: A practical approach. Oxford University Press, New York); Sutcliffe, I. C. and R. R. B. Russell. 1995. Lipoproteins of Gram-positive bacteria. J. Bacteriol. 177:1123-1128). Since the identification of the cleavage site is the most important factor in identifying putative lipoproteins, each correctly positioned amino acid contributed toward reaching the minimum score of three. (b) A hydrophobic region rich in alanine and leucine prior to the cleavage site (Pugsley, A. P.. 1993. The complete general secretory pathway in Gram-negative bacteria. Microbiol. Rev. 57:50-108) contributed toward reaching the minimum score of three.
(c) A short stretch of hydrophilic amino acids greater than or equal to 1 usually lysine or arginine following the N-terminal methionine (Pugsley, A. P.. 1993. The complete general secretory pathway in Gram-negative bacteria. Microbiol. Rev. 57:50-108) contributed toward reaching the minimum score of three.

A list of ORFs in the *Chlamydia pneumoniae* genome encoding putative lipoproteins is set forth above in the specification.

### LPS-Related ORFs of Chlamvdia pneumoniae

Lipopolysaccharide (LPS) is an important major surface antigen of Chlamydia cells. Monoclonal antibodies (Mab) directed against LPS of *Chlamydia pneumoniae* have been identified that can neutralize the infectivity of *Chlamydia pneumoniae* both in vitro and in vivo (Peterson, E.M., de la Maza, L.M., Brade, L., Brade, H. 1998. Characterization of a Neutralizing Monoclonal Antibody Directed at the Lipopolysaccharide of Chlamydia pneumonia. Infect. Immun. Aug. 66(8):3848-3855.) Chlamydial LPS is composed of lipid A and a core oligosaccharide portion and is phenotypically of the rough type (R-LPS) (Lukacova, M., Baumann, M., Brade, L., Mamat, U., Brade, H. 1994. Lipopolysaccharide Smooth-Rough Phase Variation in Bacteria of the Genus Chlamydia. Infect. Immun. June 62(6):2270-2276.) The lipid A component is composed of fatty acids which serve to anchor LPS in the outer membrane. The core component contains sugars and sugar derivatives such as a trisaccharide of 3-deoxy-D-manno-octulosonic acid (KDO) (Reeves, P.R., Hobbs, M., Valvano, M.A., Skurnik, M., Whitfield, C., Coplin, D., Kido, N., Klena, J., Maskell, D., Raetz, C.R.H., Rick, P.D. 1996. Bacterial Polysaccharide Synthesis and Gene Nomenclature pp. 10071-10078, Elsevier Science Ltd.). The KDO gene product is a multifunctional glycosyltransferase and represents a shared epitope among the Chlamydia. For a review of LPS biosynthesis see, e.g., Schnaitman, C.A., Klena, J.D. 1993. Genetics of Lipopolysaccharide Biosynthesis in Enteric Bacteria. Microbiol. Rev. 57:655-682.

A text search of the ORF blastp results identified several genes that are involved in Chlamydial LPS production with a P score better than e⁻¹⁰. The following key-terms were used in the text search: KDO, CPS (Capsular Polysaccharide Biosynthesis), capsule, LPS, rfa, rfb, rfc, rfe, rha, rhl, core, epimerase, isomerase, transferase, pyrophosphorylase, phosphatase, aldolase, heptose, manno, glucose, lpxB, fibronectin, fibrinogen, fucosyltransferase , lic, lgt, pgm, tolC, rol, ChoP, phosphorylcholine, waaF, PGL-Tb1. A list of ORFs in the *Chlamydia pneumoniae* genome encoding putative polypeptides involved in LPS biosynthesis is set forth above in the specification.

### Type III And Other Secreted Products

Type III secretion enables gram-negative bacteria to secrete and inject pathogenicity proteins into the cytosol of eukaryotic host cells (Hueck, C. J., 1998. Type III Protein Secretion Systems in Bacterial Pathogens of Animals and Plants. In Microbiology and Molecular Biology Reviews. 62:379-433.) These secreted factors often resemble eukaryotic signal transduction factors, thus enabling the bacterium to redirect host cell functions (Lee, C.A., 1997. Type III secretion systems: machines to deliver bacterial proteins into eukaryotic cells? Trends Microbiol. 5:148-156.) In an attempt to corrupt normal cellular functions, Chlamydial pathogenicity factors injected into the host cytosol will nonetheless, as cytoplasmic constituents be processed and presented in the context of the Major Histocompatibility Complex (MHC class I). As such, these pathogenicity proteins represent MHC class I antigens and will play an important role in cellular immunity. Also included in this set are secreted non-type **III** products that may play a role as vaccine components.

A text search of the ORF blastp results identified genes that are involved in *Chlamydia pneumoniae* protein secretion with a P score better than e⁻¹⁰. The following key-terms were used in the text search in an effort to identify surface localized or secreted products: Yop, Lcr, Ypk, Exo, Pcr, Pop, Ipa, Vir, Ssp, Spt, Esp, Tir, Hrp, Mxi, hemolysin, toxin, IgA protease, cytolysin, tox, hap, secreted and Mip.

*Chlamydia pneumoniae* ORFs that did not meet the above keyword search criteria, but have homologs *in Chlamydia trachomatis* that do meet the search criteria are included herein. The *Chlamydia trachomatis* genome (French patent applications FR97-15041, filed 28 November 1997 and 97-16034 filed 17 December 1997) was analyzed using the above search criteria and a number of ORFs were identified. These *Chlamydia trachomatis* ORFs were tested against the *Chlamydia pneumoniae* genome using Blastp. Any *Chlamydia pneumoniae* ORF with a Blastp P value < e⁻¹⁰ against a *Chlamydia trachomatis* homolog, identified using the above search criteria, was included. A list of ORFs in the *Chlamydia pneumoniae* genome encoding putative secreted proteins is in the specification.

### Chlamydia pneumoniae: RGD Recognition Sequence

Proteins that contain Arg-Gly-Asp (RGD) attachment site, together with integrins that serve as their receptor constitute a major recognition system for cell adhesion. The RGD sequence is the cell attachment site of a large number of adhesive extracellular matrix, blood, and cell surface proteins and nearly half of the known integrins recognize this sequence in their adhesion protein ligands. There are many RGD containing microbial proteins such as the penton protein of adenovirus, the coxsackie virus, the foot and mouth virus and pertactin, a 69 kDa (kilodalton) surface protein of *Bordetella pertussis,* that serve as ligands through which these microbes bind to integrins on the cell surfaces and gain entry into the cell. The following provides evidence supporting the importance of RGD in microbial adhesion:
a) The adenovirus penton base protein has a cell rounding activity and when penton base was expressed in E. coli, it caused cell rounding and cells adhered to polystyrene wells coated with the protein. Mutant analysis showed that both these properties required an RGD sequence. Virus mutants with amino acid substitutions in the RGD sequence, showed much less adherence to HeLa S3 cells, and also were delayed in virus reproduction (Bai, M., Harfe, B., and Freimuth, P. 1993. Mutations That Alter an RGD Sequence in the Adenovirus Type 2 Penton Base Protein Abolish Its Cell-Rounding Activity and Delay Virus Reproduction in Flat Cells. J. Virol. 67:5198-5205).
b) It has been shown that attachment and entry of coxsackie virus A9 to GMK cells were dependent on an RGD motif in the capsid protein VP1. VP1 has also been shown to bind αᵥβ₃ integrin, which is a vitronectin receptor (Roivainen, M., Piirainen, L., Hovi, T., Virtanen, I., Riikonen, T., Heino, J., and Hyypia, T. 1994. Entry of Coxsackievirus A9 into Host Cells: Specific Interactions with avb3 Integrin, the Vitronectin Receptor Virology, 203:357-65).
c) During the course of whooping cough, *Bordetella pertussis* interacts with alveolar macrophages and other leukocytes on the respiratory epithelium. Whole bacteria adheres by means of two proteins, filamentous hemagglutinin (FHA) and pertussis toxin. FHA interacts with two classes of molecules on macrophages, galactose containing glycoconjugates and the integrin CR3. The interaction between CR3 and FHA involves recognition of RGD sequence at the positions 1097-1099 in FHA (Relman, D., Tuomanen, E., Falkow, S., Golenbock, D. T., Saukkonen, K., and Wright, S. D. "Recognitition of a Bacterial Adhesin by an Integrin: Macrophage CR3 Binds Filamentous Hemagglutinin of Bordetella Pertussis." Cell, 61:1375-1382 (1990)).
d) Pertactin, a 69 kDa outer membrane protein of *Bordetella pertussis,* has been shown to promote attachment of Chinese hamster ovary cells (CHO). This attachment is mediated by recognition of RGD sequence in pertactin by integrins on CHO cells and can be inhibited by synthetic RGD containing peptide homologous to the one present in pertactin (Leininger, E., Roberts, M., Kenimer, J. G., Charles, I. G., Fairweather, N., Novotny, P., and Brennan, M. J. 1991. Pertactin, an Arg-Gly-Asp containing Bordetella pertussis surface protein that promotes adherence of mammalian cells Proc. Natl. Acad. Sci. USA, 88:345-349).
e) The RGD sequence is highly conserved in the VP1 protein of foot and mouth disease virus (FMDV). Attachment of FMDV to baby hamster kidney cells (BHK) has been shown to be mediated by VP1 protein via the RGD sequence. Antibodies against the RGD sequence of VP1 blocked attachment of virus to BHK cells (Fox, G., Parry. N. R., Barnett, P. V., McGinn, B., Rowland, D. J., and Brown, F. 1989. The Cell Attachment Site on Foot-and-Mouth Disease Virus Includes the Amino Acid Sequence RGD (Arginine-Glycine-Aspartic Acid) J. Gen. Virol., 70:625-637).

It has been demonstrated that bacterial adherence can be based on interaction of a bacterial adhesin RGD sequence with an integrin and that bacterial adhesins can have multiple binding site characteristic of eukaryotic extracellular matrix proteins. RGD recognition is one of the important mechanisms used by microbes to gain entry into eukaryotic cells.

The complete deduced protein sequence of the *Chlamydia pneumoniae* genome was searched for the presence of RGD sequence. There were a total of 54 ORFs that had one or more RGD sequences. Not all RGD containing proteins mediate cell attachment. It has been shown that RGD containing peptides that have proline immediately following the RGD sequence are inactive in cell attachment assays (Pierschbacher & Ruoslahti. 1987. Influence of stereochemistry of the sequence Arg-Gly-Asp-Xaa on binding specificity in cell adhesion. J. Biol. Chem. 262:17294-98). ORFs that had RGD, with proline as the amino acid following the RGD sequence were excluded from the list. Also, RGD sequence may not be available at the surface of the protein or may be present in a context that is not compatible with integrin binding. Since not all RGD- containing proteins are involved in cell attachment, several other criteria were used to refine the list of RGD- containing proteins. A list of ORFs in the *Chlamydia pneumoniae* genome encoding polypeptides with RGD recognition sequence(s) is in the specification.

### Non-Chlamydia trachomatis ORFs

*Chlamydia pneumoniae* ORFs were compared to the ORFs in the *Chlamydia trachomatis* genome (French patent applications FR97-15041, filed 28 November 1997 and 97-16034 filed 17 December 1997) using Blastp. Any *Chlamydia pneumoniae* ORF with a Blastp P value worse than e⁻¹⁰ (i.e. >e⁻¹⁰) against *Chlamydia trachomatis* ORFs are included in this section. A list of ORFs in the *Chlamydia pneumoniae* genome which are not found in *Chlamydia trachomatis* is set forth above in the specification.

### Cell Wall Anchor Surface ORFs

Many surface proteins are anchored to the cell wall of Gram-positive bacteria via the conserved LPXTG motif (Schneewind, O., Fowler, A., and Faull, K.F. 1995. Structure of the Cell Wall Anchor of Surface Proteins in Staphylococcus aureus. Science 268:103-106). A search of the *Chlamydia pneumoniae* ORFs was done using the motif LPXTG. A list of ORFs in the *Chlamydia pneumoniae* genome encoding polypeptides anchored to the cell wall is in the specification.

### ATCC Deposits

Samples of *Chlamydia pneumoniae* were deposited with the American Type Culture Collection (ATCC), Rockville, Maryland, on November 19, 1998 and assigned the accession number 1366-VR. Cells can be grown, harvested and purified, and DNA can be prepared as discussed above. In order to enable recovery of specific fragments of the chromosome, one can run targeted PCR reactions, whose amplification products can then be sequenced and/or cloned into any suitable vector, according to standard procedures known to those skilled in the art.

In addition, a sample of three pools of clones covering chromosomal regions of interest were deposited with the American Type Culture Collection (ATCC), Rockville, Maryland, on November 19, 1998. Each pool of clones contains a series of clones. When taken together, the three pools in the sample cover a portion of the chromosome, with a redundancy of slightly more than two. The total number of clones in the sample is 196.

The clones cover the following three regions of interest:
(i) position 30,000 to 40,000 of SEQ ID No. 1, referred to as region A;
(ii) position 501,500 to 557,000 of SEQ end No. 1, referred to as region B; and
(iii) position 815,000 to 830,000 of SEQ ID No. 1, referred to as region C.

Table 4 lists group of oligonucleotides to be used to amplify ORF 291 according to standard procedures known to those skilled in the art. Such oligonucleotides are listed as SEQ ID Nos.1936, 1957, 4460 and 4461. For each ORF, the following is listed: one forward primer positioned 2,000 bp upstream of the beginning of the ORF; one forward primer positioned 200 bp upstream of the beginning of the ORF; one reverse primer positioned 2,000 bp downstream at the end of ORF, which is 2,000 bp upstream of the end site of the ORF on the complementary strand; and one reverse primer 200 bp downstream at the end of ORF, which is 200 bp upstream of the end site of the ORF on the complementary strand. The corresponding SEQ ID Nos. for the primers are listed in Table 4, where Fp is the proximal forward primer; Fd is the distal forward primer; Bp is the proximal reverse primer; and Bd is the distal reverse primer. The positions of the 5' ends of each of these primers on the nucleotide sequence of SEQ ID No. I are shown in Table 5.

**TABLE 1**

| **ORF** | **Begin** | **End** | **Homology** | **ID** | **Species** | **Score** | **1%** |
|---|---|---|---|---|---|---|---|
| ORF291 | 323190 | 322366 | outer membrane protein | AE000654 | *Helicobacter pylori* | 376 | 43 |

**Table 2**

| **ORF Nos.** | **begin** | **end** | **potential start** |
|---|---|---|---|
| 291 | 323190 | 322366 | 323181 |

**Table 4**

| ***SEQ ID NO (ORF)*** | ***Fp*** | ***Fd*** | ***Bp*** | ***Bd*** |
|---|---|---|---|---|
| 291 | 1956 | 1957 | 4460 | 4461 |

**TABLE 5**

| ***SEQ ID*** | ***or.*** | ***5'position*** |
|---|---|---|
| 1956 | F | 322084 |
| 1957 | F | 320217 |

### Publications Cited in the Specification

Adames et al., 1985, Nature, 318 : 533-538.
Aldous, M.B. et al., 1992, J. Infect. Dis., 166 : 646-649.
Alexander et al., 1987, Mol. Cell. Biol., 7 : 1436-1444.
Allan, G. M. et al., 1995, Vet. Microbiol., 44 : 49-64.
Altschul, S.F. et al., 1990, J. Mol. Biol., 215 : 403-410.
Altschul et al., 1993, Nature Genetics, 3 : 266-272.
Altschul et al.,. 1997, Nucl. Acids Res., 25 : 3389-3402.
Ansubel et al., 1989, Current Protocols in Molecular Biology,
Arlinghaus, H.F. et al., 1997, Anal. Biochem., 69, 18, 3747-53.
Bai, M. Et al., 1993, J. Virol., 67 : 5198-5205.
Barany, F., 1911, PNAS. USA, 88 : 189-193.
Beattie, K. et al., 1993, Clin. Chem., 39(4): 719-721.
Bemoist and Chambon, 1981, Nature, 290 : 304-310.
Borman, S., 1996, Chem. Eng. News, 74(50): 42-43.
Braun, J. et al., 1994 Ann., Rheum Dis 53 : 100-105.
Brinster et al., 1982, Nature, 296 : 39-42.
Buckholz, R.G., 1993, Yeast systems for the expression of heterologous gene products. Curr. Op. Biotechnology 4 : 538-542.
Burg, J.L. et al., 1996, Mol. and Cell. Probes, 10 :257-271.
Campbell, LA. et al., 1992 J. Clin. Microbiol. 30 : 434-439.
Casas-Ciria J. et al., 1996
Chatelier, R.C. et al., 1995, Anal. Biochem., 229, 1, 112-118.
Chee, M. et al.,1996, Science, 274: 610-613.
Chu, B.C.F. et al., 1986, NAR, 14 : 5591-5603.
Chu, P.W.G. et al., 1993, Virus Research, 27 : 161-171.
Clark, E.G., 1997, American Association of Swine Practitioners, 499-501. Cole et al., 1985, in Monoclonal Antibodies and Cancer Therapy, Alan R. Liss, Inc., pp. 77-96.
Cote et al., 1983, PNAS USA, 80 : 2026-2030.
Cserzo, M., Wallin, E., Simon, I. von Heijne G and Elofsson, A., 1997, Prot. Eng., 10 : 673-676.
DeBoer et al., 1980, Scientific American, 242 : 74-94.
DeBoer et al., 1983, PNAS USA, 80 : 21-25.
Derisi, J. et al., 1996, Nature Genet, 14 : 457-460.
Distance Relationships: Atlas of Protein Sequence and Structure, Washington : National Biomedical Reserach Foundation.
Duck, P. et al., 1990, Biotechniques, 9: 142-147.
Dulac, G.C. et al., 1989, Can. J. Vet. Res., 53 : 431-433.
Edwards, C.P., and Aruffo, A., 1993, Current applications of COS cell based transient expression systems. Curr. Op. Biotechnology 4 : 558-563.
Edwards, S. et al., 1994, Vet. Rec., 134 : 680-681.
Erlich, H.A., 1989, In PCR Technology. Principles and Applications for DNA Amplification. New York: Stockton Press.
Falsey, et al., J. Infect. Dis. 168 :493-496. Fanger and Drakeman, 1995, Drug News and Perspectives, 8 : 133-137. Felgner, et al., 1987, Proc. Natl. Acad. Sci., 84 : 7413.
Fodor, S.P.A. et al., 1991, Science, 251 : 767-771.
Fontes, E.P.B. et al., 1994, J. Biol. Chem., Vol. 269, N°11 : 8459-8465.
Fox, G. Et al., 1989, J. Gen. Virol., 70 : 625-637.
Fraley et al., 1980, J. Biol. Chem., 255 : 10431.
Gardner et al., 1981, Nucl. Acids Res. 9 : 2871
Gaydos, C.A. et al., 1994 J. Clin. Microbiol. 32 : 903-905.
Grayston, J.T. et al., 1986 N. Engl. J. Med., 315 : 161-168.
Grayston, JT. et al., 1996 Rev., Med Interne 17, 45S-47S.
Gonnet et al., Science, 256 : 1443-1445. Green Publishing Associates and Wiley Interscience, N.Y. Pearson and Lipman, 1988, PNAS USA, 85(8) : 2444-2448.
Grosschedl et al., 1984, Cell, 38 : 647-658.
Guateli, J.C. et al., 1990, PNAS. USA, 87 : 1874-1878.
Hackland, A.F. et al., 1994, Arch. Virol., 139 : 1-22.
Hahn, DL. Et al., 1991 JAMA. 266
Haidl, et al., 1992 N. Engl. J. Med. 326:576-577.
Haidl, et al., Chlamydial infections 1994
Hammer et al., 1987, Science, 235 : 53-58.
Hanahan, 1985, Nature, 315 : 115-122.
Hanson, S.F. et al., 1995, Virology, 211 : 1-9.
Harding, J.C., 1997, American Association of Swine Practitioners, 503.
Harding, R.M. et al., 1993, Journal of General Virology, 74 : 323-328.
Hashiguchi, K. et al., 1992 J. Laryngol. Otol. 106 : 208-210.
Hayashi, S. and Wu, H.C., 1992, in N.M. Hooper and AJ. Turner (ed.) Lipid Modification of Proteins: A Practical Approach. Oxford University Press, New York, pp. 261-285.
Heinkoff and Heinkoff, 1993, Proteins, 17 : 49-61.
Herrera-Estrella et al., 1983, Nature, 303 : 209-213.
Herrera-Estrella, 1984, Nature, 310: 115-120.
Heyraud-Nitschke, F. et al., 1995, Nucleic Acids Research, Vol. 23, N° 6. Higgins et al., 1996, Meth. Enzymol., 266 : 383-402.
Homer, G.W., 1991, Surveillance 18(5) : 23.
Houbenweyl, 1974, in Meuthode der Organischen Chemie, E. Wunsch Ed., Volume 15-I et 15-II, Thieme, Stuttgart.
Hueck, C.J., 1998, Molec. Biology Rev., 62 : 379-433.
Huovinen, P. et al., 1989 Ann., Intern Med 110:612-616.
Huse et al., 1989, Science, 246 : 1275-1281.
Huygen, K. et al., 1996, Nature Medicine, 2(8) : 893-898.
Innis, M.A. et al. 1990. in PCR Protocols. A guide to Methods and Applications. San Diego : Academic Press.
Inoue et al., 1987, Nucl. Acids Res., 15 : 5131-6148.
Inoue et al., 1987, FEBS Lett. 215 : 327-330.
Jackson, LA. et al., 1997 Am., J. Pathol. 150.: 1785-1790.
Jantos et al., 1997, J. Clin. Microbiol., 35(3) : 620-623.
Kabat E. Et al., 1983, Sequences of Proteins of Immunological Interest, U.S. Dept. Of Health and Human Services.
Kaneda, et al., 1989, Science, 243 : 375.
Kelsey et al., 1987, Genes and Devel., 1 : 161-171.
Kievitis, T. et al., 1991, J. Virol. Methods. 35 : 273-286.
Kleemola, M. et al., 1988, J. Infect. Dis. 157 : 230-236.
Kohler, G. et al., 1975, Nature, 256(5517) : 495-497.
Kollias et al., 1986, Cell, 46 : 89-94.
Kozbor et al., 1983, Immunol. Today, 4 : 72.
Krone, J.R. et al., 1997, Anal. Biochem., 244, 1,124-132.
Krumlauf et al., 1985, Mol. Cell. Biol., 5 : 1639-1648.
Kuo, CC. et al., 1988, J. Clin. Microbiol. 26: 812-815.
Kuo, CC. et al., 1993, J. Infect. Dis. 167 : 841-849.
Kwoh, D.Y. et al., 1989, PNAS. USA, 86: 1173-1177.
Ladany, S. et. al., 1989, J. Clin. Microbiol. 27 : 2778-2783.
Laitinen, K. et al., 1997. Chlamydia pneumoniae Infection Induces Inflammatory Changes in the Aortas of Rabbits. Infect. Immun. 65:4832-4835.
Lazarowitz, S. G. et al., 1989, The EMBO Journal, Vol. 8 N° 4 : 1023-1032.
Leder et al., 1986, Cell, 45 : 485-495.
Lee, C.A., 1997, Trends Microbiol., 5 : 148-156.
Leininger, E. et al., 1991, PNAS USA, 88 : 345-349.
Lipshutz, R.J. et al., 1995, Biotechniques, 19(3) : 442-447.
Liu, H. et al., 1997, J. Gen. Virol. 78(Pt6) : 1265-1270.
Livache, T. et al., 1994, NAR, 22(15) : 2915-2921.
Lockhart, D.J. et al., 1996, Nature Biotechnol., 14 : 1675-1680.
Longbottom et al., 1998, Infect Immunol., 66 : 1317-1324.
Luckow, V.A., 1993, Baculovirus systems for the expression of human gene products. Curr. Op. Biotechnology 4: 564-572.
Lukacova, M. Et al., 1994, Infect Immunol. June, 62(6): 2270-2276.
MacDonald, 1987, Hepatology, 7 : 425-515.
Mankertz, A. et al., 1997, J. Virol., 71 : 2562-2566.
Mason et al., 1986, Science, 234 : 1372-1378.
Matson, R.S. et al., 1994, Analytical Biochemistry, 217 : 306-310.
Matthews, J.A. et al., 1988, Anal. Biochem., 169 : 1-25.
McNeilly, F. et al., 1996, Vet. Immunol. Immunopathol., 49 : 295-306.
Meehan, B.M. et al., 1997, J. Gen. Virol., 78 : 221-227.
Mérel, P., 1994, De la PCR aux puces à ADN, Biofutur, 139 : 58.
Merrifield, R.D., 1966, J. Am. Chem. Soc., 88(21) : 5051-5052.
Midoux, 1993, Nucleic Acids Research, 21 : 871-878.
Miele, E.A. et al., 1983, J. Mol. Biol., 171 : 281-295.
Moazed, T.C. et al., 1997. Murine Model of Chlamydia pneumoniae Infection and Atherosclerosis. J. Infect. Dis. 175:883-890.
Mogram et al., 1985, Nature, 315 : 338-340.
Mordhorst, C.H. et al., 1992 Eur., J. Clin. Microbiol. Infect Dis 11 : 617-620.
Morrison et al., 1984, PNAS USA, 81:6851-6855.
Morrison, R.P. et al., 1995. Gene Knockout Mice Establish a Primary Protective Role for Major Histocompatibility Complex Class II-Restricted Responses in Chlamydia trachomatis. Infect. Immun. 63:4661-4668.
Murphy, F.A. et al., 1995, Sixth Report of the International Committee on Taxonomy of Viruses. Springer-Verlag Wien New York.
Nakai, K. and Kanehisa, M., 1991, Proteins, 11 : 95-110.
Nielsen, H. et al., 1997, Protein Engin., 10: 1-6.
Neuberger et al., 1984, Nature, 312 : 604-608.
O'Donell-Maloney, M.J., 1996, Trends Biotechnol., 14 : 401-407.
Ogawa, H. et al., 1992 J. Laryngol. Oto 106 : 490-492.
Olins, P.O., and Lee, S.C., 1993, Recent advances in heterologous gene expression in E. coli. Curr. Op. Biotechnology 4 : 520-525.
Omitz et al., 1986, Cold Spring Harbor Symp. Quant. Biol., 50 : 399-409. Pagano et al., 1967, J. Virol., 1 : 891.
Peterson, E.M. et al., 1998, Infect. Immunol. Aug., 66(8) : 3848-3855.
Peterson, E. et al., 1988. Protective Role of Magnesium in the Neutralization by Antibodies of Chlamydia trachomatis Infectivity.
Pierschbacher and Ruoslahti, 1987, J. Biol. Chem., 262 : 17294-17298.
Pinkert et al., 1987, Genes and Devel., 1 : 268-276.
Pugsley, A.P., 1993, Microbiol. Rev., 57 : 50-108.
Puolakkainen, M. et al., 1993 J. Clin. Microbiol. 31 : 2212-2214.
Rank, R.G. et al., 1988. Susceptibility to reinfection after a primary chlamydial genital infection. Infect. Immun. 56:2243-2249.
Readhead et al., 1987, Cell, 48 : 703-712.
Reeves, P.R. et al., 1996, in Bacterial Polysaccharide Synthesis and Gene Nomenclature, Elsevier Science Ltd., pp. 10071-10078.
Roivainen, M. Et al., 1994, Virology, 203 : 357-365. Rolfs, A. et al., 1991, In PCR Topics. Usage of Polymerase Chain reaction in Genetic and Infectious Disease. Berlin : Springer-Verlag.
Salzberg et al., 1998, Nucl. Acids Res., 26 : 544-548.
Sambrook, J. et al., 1989, In Molecular cloning: A Laboratory Manual. Cold Spring Harbor, NY : Cold Spring Harbor Laboratory Press.
Sanchez-Pescador, R., 1988, J. Clin. Microbiol., 26(10): 1934-1938.
Sani, 1985, Nature, 314 : 283-286.
Sarver et al., 1990, Science, 247 : 1222-1225.
Schachter, J. 1980. Chlamydiae, p.357-365. In E.H. Lennette (ed.), Manual of clinical microbiology, 3rd ed. American Society for Microbiology, Washington, D.C.
Schneewind, O. Et al., 1995, Science, 268: 103-106.
Schwartz and Dayhoff, eds., 1978, Matrices for Detecting Karlin and Altschul, 1990, PNAS USA, 87 : 2267-2268.
Segev D., 1992, in « Non-radioactive Labeling and Detection of Biomolecules ». Kessler C. Springer Verlag, Berlin, New-York : 197-205.
Sheldon, E.L., 1993, Clin. Chem., 39(4) : 718-719.
Shiver, J.W., 1995, in Vaccines 1995, eds Chanock, R.M. Brown, F. Ginsberg, H.S. & Norrby, E.), pp.95-98, Cold Spring Harbor Laboratory Press, Cold Spring Harbor, New York.
Shoemaker, D.D. et al., 1996, Nature Genet, 14 : 450-456.
Shor, A. et. al.., 1992 S. Afr. Med. J. 82 : 158-161.
Sosnowsky et al., 1997, PNAS., 94, 1119-1123.
Struyve, M. et al., 1991, J. Mol. Biol., 218 : 141-148.
Sundelof, et al., 1993 Scand. J. Infec. Dis. 25:259-261.
Sutcliffe, I.C. and Russell, R.R.B., 1995, J. Bacteriol. 177 : 1123-1128.
Swift et al., 1984, Cell, 38 : 639-646.
Takeda et al., 1985, Nature, 314 : 452-454.
Tascon, R.E et al., 1996, Nature Medicine, 2(8) : 888-892.
Thom, D.H. et al., 1990 Am. J. Epidemiol 132 : 248-256.
Thomas, GN. et al., 199? Scand., J. Infect. Dis. Suppl 104, 30-33.
Tischer, I. et al., 1982, Nature, 295 : 64-66.
Tischer, I. et al., 1986, Arch. Virol., 91 : 271-276.
Tischer, I. et al., 1988, Zentralbl Bakteriol Mikrobiol Hyg [A] 270 : 280-287.
Tischer, I. et al., 1995, Arch. Virol., 140 : 737-743.
Tompson et al., 1994, Nucl. Acids Res., 22(2) : 4673-4680.
Urdea, M.S., 1988, Nucleic Acids Research, 11 : 4937-4957.
Villa-Kamaroff et al., 1978, PNAS USA, 75 : 3727-3731.
Wagner et al., 1981, PNAS USA, 78 : 1441-1445.
Walker, G.T. et al., 1992, NAR 20: 1691-1696.
Walker, G.T. et al., 1992, PNAS. USA, 89 : 392-396.
White, B.A. et al., 1997, Methods in Molecular Biology, 67, Humana Press, Towota.
Yamamoto et al., 1980, Cell, 22 : 787-797.
Yershov, G. et al., 1996, PNAS., USA, 93 : 4913-4918.

### SEQUENCE LISTING

<110>SERONO GENETICS INSTITUTE SA
   <120>Chlamydia pneumoniae outer membrane polypeptide, fragments thereof and uses thereof, in particular for the diagnosis, prevention and treatment of infection
   <151>1997-11-21
   <160>6
<210>ORF 291
   <211>825
   <212>DNA
   <213>Chlamydia pneumoniae
   <400>ORF291
<220>
   <223> begin: position nt323190, end: position nt322366 (see Table 2) in the complete genome sequence SEQ ID No.1 disclosed in the parent patent application WO99/2710 
accat tgtcttgagt 322380
cattgtcaaa atattcccat gatattttgt atcaaaaaaa tgttgtacag aaggagattg 322440
aaacagcttc tgtaatttta tcattttagg agaacctacg tcttcagaac gaatgacaac 322500
aaggtttgta tacttagata ccgaaagatc ctctaaacaa agactatctt tctttggaga 322560
aaggtttgct gctatagcaa aatttccagg aatgacagca gcatcaacgt caggaagaga 322620
tccgacaaga agaggagctg acacctctaa tatgttgata cttctatttt ctttcccaca 322680
gacatcttta gctgtcatat ttaaattagc aggccctttg caaacaatga gtccgcactc 322740
ttctaacaag tgtagagcac gctgagcatt cgtacgatcc acaggaatcg ctatagtcag 322800
tttcttctgg ctttttaagc gctctaaaga agaatgtttc ttagaataaa ttgcttgagg 322860
ttccaaatga actttagcga taacaactaa ttcaccctta caatcataac gctcgcattc 322920
gtcatcaaga aaagcttgat gttgaaagta atttgcatct acttgtttat ccaaaagcaa 322980
acgattagga atacgataat catctactgg aagtattttc agcttgattc caagatcttt 323040
agcctcttcc tgtaaactct ccaataattc cgcatgaggt gtcggactgg ctacaatacg 323100
tattttattc tgagcatctt ccttatggca agaactcaaa acaaaaatta aacctacaag 323160
taatgataat ttttttttca tagaatccct
<210>SEQ ID No.291
   <211>275
   <212>PRT
   <213>Chlamydia pneumoniae
   <400> SEQ ID No.291 
Arg Asp Ser Met Lys Lys Lys Leu Ser Leu Leu Val Gly Leu Ile Phe
1               5                   10                  15
Val Leu Ser Ser Cys His Lys Glu Asp Ala Gln Asn Lys Ile Arg Ile
            20                  25                  30
Val Ala Ser Pro Thr Pro His Ala Glu Leu Leu Glu Ser Leu Gln Glu
        35                  40                  45
Glu Ala Lys Asp Leu Gly Ile Lys Leu Lys Ile Leu Pro Val Asp Asp
    50                  55                  60
Tyr Arg Ile Pro Asn Arg Leu Leu Leu Asp Lys Gln Val Asp Ala Asn
65                  70                  75                  80
Tyr Phe Gln His Gln Ala Phe Leu Asp Asp Glu Cys Glu Arg Tyr Asp
                85                  90                  95
Cys Lys Gly Glu Leu Val Val Ile Ala Lys Val His Leu Glu Pro Gln
            100                 105                 110
Ala Ile Tyr Ser Lys Lys His Ser Ser Leu Glu Arg Leu Lys Ser Gln
        115                 120                 125
Lys Lys Leu Thr Ile Ala Ile Pro Val Asp Arg Thr Asn Ala Gln Arg
    130                 135                 140
 
Ala Leu His Leu Leu Glu Glu Cys Gly Leu Ile Val Cys Lys Gly Pro
145                 150                 155                 160
Ala Asn Leu Asn Met Thr Ala Lys Asp Val Cys Gly Lys Glu Asn Arg
                165                 170                 175
Ser Ile Asn Ile Leu Glu Val Ser Ala Pro Leu Leu Val Gly Ser Leu
            180                 185                 190
Pro Asp Val Asp Ala Ala Val Ile Pro Gly Asn Phe Ala Ile Ala Ala
        195                 200                 205
Asn Leu Ser Pro Lys Lys Asp Ser Leu Cys Leu Glu Asp Leu Ser Val
    210                 215                 220
Ser Lys Tyr Thr Asn Leu Val Val Ile Arg Ser Glu Asp Val Gly Ser
225                 230                 235                 240
Pro Lys Met Ile Lys Leu Gln Lys Leu Phe Gln Ser Pro Ser Val Gln
                245                 250                 255
His Phe Phe Asp Thr Lys Tyr His Gly Asn Ile Leu Thr Met Thr Gln
            260                 265                 270
Asp Asn Gly
        275
<210> SEQ ID No.1956
   <211>20
   <212>DNA
   <400> SEQ ID No.1956
   cctcagcata agtgctacta 20
<210> SEQ ID No.1957
   <211>20
   <212>DNA
   <400> SEQ ID No.1957
   ctccttggcg tgtaggaata 20
<210> SEQ ID No.4460

   <211>20
   <212>DNA
   <400> SEQ ID No.4460
   ctgaaagcta tccacagctc 20
<210> SEQ ID No.4461
   <211>20
   <212>DNA
   <400> SEQ ID No.4461
   ccagggattt gtcaaggaac 20

## Claims

1. An isolated polynucleotide having a nucleotide sequence of an open reading frame (ORF) of a *Chlamydia pneumoniae* genome, comprising:
(a) the nucleotide sequence ORF291 encoding the amino acid sequence SEQ ID N0: 291;
(b) a nucleotide sequence exhibiting at least 80% identity with the nucleotide sequence ORF291; or
(c) a complementary sequence thereof.

2. The polynucleotide of Claim 1, which encodes the following polypeptides or fragments thereof:
(a) a *Chlamydia pneumoniae* polypeptide of the cellular envelope, preferably of the outer cellular envelope;
(b) a *Chlamydia pneumoniae* transmembrane polypeptide having between 1 and 3 transmembrane domains;
(c) a *Chlamydia pneumoniae* surface exposed polypeptide;
(d) a *Chlamydia pneumoniae* lipoprotein; or
(e) a *Chlamydia pneumoniae* polypeptide that is not found in *Chlamydia trachomatis.*

3. A polynucleotide encoding a fusion protein, comprising the polynucleotide of Claim 1 or 2, ligated in frame to a polynucleotide encoding a heterologous polypeptide.

4. A recombinant vector that contains the polynucleotide of Claims 1 to 3.

5. A recombinant vector that contains the polynucleotide of Claims 1 to 3, operatively associated with a regulatory sequence that controls gene expression.

6. A genetically engineered host cell that contains the polynucleotide of Claims 1 to 3.

7. A genetically engineered host cell that contains the polynucleotide of Claims 1 to 3 operatively associated with a regulatory sequence that controls gene expression in the host cell.

8. A method for producing a polypeptide, comprising:
(a) culturing the genetically engineered host cell of Claim 6 or 7 under conditions suitable to produce the polypeptide encoded by the polynucleotide; and
(b) recovering the polypeptide from the culture.

9. A polypeptide encoded by the polynucleotide of Claims 1 to 3.

10. The polypeptide of Claim 9 which comprises the following polypeptides or fragments thereof:
(a) a *Chlamydia pneumoniae* polypeptide of the cellular envelope, preferably of the outer cellular envelope;
(b) a *Chlamydia pneumoniae* transmembrane polypeptide having between 1 and 3 transmembrane domains;
(c) a *Chlamydia pneumoniae* surface exposed polypeptide;
(d) a *Chlamydia pneumoniae* lipoprotein; or
(e) a *Chlamydia pneumoniae* polypeptide that is not found in *Chlamydia trachomatis.*

11. A fusion protein encoded by the polynucleotide of Claim 9 or 10.

12. The polypeptide of Claims 9 to 11 which immunoreacts with seropositive serum of an individual infected with *Chlamydia pneumoniae.*

13. An antibody that immunospecifically binds to the polypeptide of Claim 9 or 10.

14. A method for the detection and/or identification of *Chlamydia pneumoniae* in a biological sample, comprising:
(a) contacting the sample with a polynucleotide primer which hybridizes specifically with the the polynucleotide ORF291, or complementary sequence thereof, in the presence of a polymerase enzyme and nucleotides under conditions which permit primer extension; and
(b) detecting the presence of primer extension products in the sample in which the detection of primer extension products indicates the presence of *Chlamydia pneumoniae* in the sample.

15. A method for the detection and/or identification of *Chlamydia pneumoniae* in a biological sample, comprising:
(a) contacting the sample with a polynucleotide probe which hybridizes specifically with the the polynucleotide ORF291, or complementary sequence thereof, under conditions which permit hybridization of complementary base pairs; and
(b) detecting the presence of hybridization complexes in the sample in which the detection of hybridization complexes indicates the presence of *Chlamydia pneumoniae* in the sample.

16. A method for the detection and/or identification of *Chlamydia pneumoniae* in a biological sample, comprising:
(a) contacting the sample with the antibody of Claim 13 under conditions suitable for the formation of immune complexes; and
(b) detecting the presence of immune complexes in the sample, in which the detection of immune complexes indicates the presence of *Chlamydia pneumoniae* in the sample.

17. A method for the detection and/or identification of antibodies to *Chlamydia pneumoniae* in a biological sample, comprising:
(a) contacting the sample with a polypeptide of Claims 9 and 10 under conditions suitable for the formation of immune complexes; and
(b) detecting the presence of immune complexes in the sample, in which the detection of immune complexes indicates the presence of *Chlamydia pneumoniae* in the sample.

18. A DNA chip containing an array of polynucleotides comprising at least one of the polynucleotides of Claims 1 to 3.

19. A protein chip containing an array of polypeptides comprising at least one of the polypeptides of Claim 9 or 10.

20. An immunogenic composition comprising the polypeptide of Claims 9 to 12 and a pharmaceutically acceptable carrier.

21. A DNA immunogenic composition comprising the expression vector of Claim 4 or 5.

22. The DNA composition of Claim 21, wherein the DNA composition directs the expression of a neutralizing epitope of *Chlamydia pneumoniae.*

23. A pharmaceutical composition comprising the polypeptide of Claims 9 to 12, or an antibody of Claim 13 and a pharmaceutically acceptable carrier.

24. Composition for the treatment or the prevention of an infection by *Chlamydia pneumoniae,* said composition comprising:
a) a polynucleotide of Claims I to 3;
b) a polypeptide of Claims 9 to 12;
c) an antibody of Claim 13;
d) a vector of Claims 4 and 5; or
e) a host cell of Claims 6 and 7.

25. A composition of Claim 24 as a vaccine.

26. A screening assay, comprising:
(a) contacting a test compound with an isolated polynucleotide of Claims 1 to 3 or with an isolated polypeptide of Claims 9 and 10; and
(b) detecting whether binding occurs.

27. A kit comprising a container containing an isolated polynucleotide of Claims 1 to 3 or a primer or a probe capable to specifically hybridizing with a polynucleotide of Claims 1 to 3, an isolated polypeptide of Claims 9 and 10, or an antibody of Claim 13.

## Patentansprüche

1. Isoliertes Polynukleotid mit einer Nukleotidsequenz eines offenen Leserasters (ORF) eines *Chlamydia-pneumoniae-Genoms,* wobei das Polynukleotid Folgendes umfasst:
(a) die Nukleotidsequenz ORF291, die die Aminosäuresequenz SEQ ID NO: 291 codiert;
(b) eine Nukleotidsequenz, die mindestens 80 % Identität mit der Nukleotidsequenz ORF291 zeigt; oder
(c) eine komplementäre Sequenz davon.

2. Polynukleotid nach Anspruch 1, das die folgenden Polypeptide oder Fragmente davon codiert:
(a) ein *Chlamydia*-*pneumoniae*-Polypeptid der Zellhülle, vorzugsweise der äußeren Zellhülle;
(b) ein *Chlamydia-pneumoniae-*Transmembranpolypeptid mit zwischen 1 und 3 Transmembrandomänen;
(c) ein oberflächenexponiertes *Chlamydia-pneumoniae*-Polypeptid;
(d) ein *Chlamydia*-*pneumoniae*-Lipoprotein oder
(e) ein *Chlamydia*-*pneumoniae*-Polypeptid, das nicht in *Chlamydia trachomatis* vorgefunden wird.

3. Polynukleotid, das ein Fusionsprotein codiert, das das Polynukleotid nach Anspruch 1 oder 2 umfasst, das "in frame" an ein Polynukleotid ligiert ist, das ein heterologes Polypeptid codiert.

4. Rekombinanter Vektor, der das Polynukleotid nach den Ansprüchen 1 bis 3 enthält.

5. Rekombinanter Vektor, der das Polynukleotid nach den Ansprüchen 1 bis 3 enthält, das operativ mit einer Regulationssequenz vereinigt ist, die die Genexpression steuert.

6. Gentechnisch manipulierte Wirtszelle, die das Polynukleotid nach den Ansprüchen 1 bis 3 enthält.

7. Gentechnisch manipulierte Wirtszelle, die das Polynukleotid nach den Ansprüchen 1 bis 3 enthält, das operativ mit einer Regulationssequenz verknüpft ist, die die Genexpression in der Wirtszelle steuert.

8. Verfahren zur Herstellung eines Polypeptids, wobei das Verfahren Folgendes umfasst:
(a) Kultivieren der gentechnisch manipulierte Wirtszelle nach Anspruch 6 oder 7 unter Bedingungen, die zur Herstellung des Polypeptids geeignet sind, das von dem Polynukleotid codiert wird; und
(b) Gewinnen des Polypeptids aus der Kultur.

9. Polypeptid, das von dem Polynukleotid nach den Ansprüchen 1 bis 3 codiert wird.

10. Polypeptid nach Anspruch 9, das die folgenden Peptide oder Fragmente davon umfasst:
(a) ein *Chlamydia-pneumoniae*-Polypeptid der Zellhülle, vorzugsweise der äußeren Zellhülle;
(b) ein *Chlamydia-pneumoniae-*Transmembranpolypeptid mit zwischen 1 und 3 Transmembrandomänen;
(c) ein oberflächenexponiertes *Chlamydia-pneumoniae*-Polypeptid;
(d) ein *Chlamydia-pneumoniae-*Lipoprotein oder
(e) ein *Chlamydia-pneumoniae*-Polypeptid, das nicht in *Chlamydia trachomatis* vorgefunden wird.

11. Fusionsprotein, das von dem Polynukleotid nach Anspruch 9 oder 10 codiert wird.

12. Polypeptid nach den Ansprüchen 9 bis 11, das mit seropositivem Serum einer Person, die mit *Chlamydia pneumoniae* infiziert ist, immunreagiert.

13. Antikörper, der immunspezifisch an das Polypeptid nach Anspruch 9 oder 10 bindet.

14. Verfahren zum Nachweis und/oder zur Identifizierung von *Chlamydia pneumoniae* in einer biologischen Probe, wobei das Verfahren Folgendes umfasst:
(a) Inkontaktbringen der Probe mit einem Polynukleotid-Primer, der spezifisch mit dem Polynukleotid ORF291 oder einer komplementären Sequenz davon hybridisiert, in Gegenwart eines Polymeraseenzyms und von Nukleotiden unter Bedingungen, die eine Primerextension ermöglichen; und
(b) Nachweisen des Vorliegens von Primerextensionsprodukten in der Probe, wobei der Nachweis von Primerextensionsprodukten das Vorliegen von *Chlamydia pneumoniae* in der Probe anzeigt.

15. Verfahren zum Nachweis und/oder zur Identifizierung von *Chlamydia pneumoniae* in einer biologischen Probe, wobei das Verfahren Folgendes umfasst:
(a) Inkontaktbringen der Probe mit einer Polynukleotid-Sonde, die spezifisch mit dem Polynukleotid ORF291 oder einer komplementären Sequenz davon hybridisiert, unter Bedingungen, die eine Hybridisierung komplementärer Basenpaare ermöglichen;
und
(b) Nachweisen des Vorliegens von Hybridisierungskomplexen in der Probe, wobei der Nachweis von Hybridisierungskomplexen das Vorliegen von *Chlamydia pneumoniae* in der Probe anzeigt.

16. Verfahren zum Nachweis und/oder zur Identifizierung von *Chlamydia pneumoniae* in einer biologischen Probe, wobei das Verfahren Folgendes umfasst:
(a) Inkontaktbringen der Probe mit dem Antikörper nach Anspruch 13 unter Bedingungen, die zur Bildung von Immunkomplexen geeignet sind; und
(b) Nachweisen des Vorliegens von Immunkomplexen in der Probe, wobei der Nachweis von Immunkomplexen das Vorliegen von *Chlamydia pneumoniae* in der Probe anzeigt.

17. Verfahren zum Nachweis und/oder zur Identifizierung von Antikörpern zu *Chlamydia pneumoniae* in einer biologischen Probe, wobei das Verfahren Folgendes umfasst:
(a) Inkontaktbringen der Probe mit einem Polypeptid nach den Ansprüchen 9 und 10 unter Bedingungen, die zur Bildung von Immunkomplexen geeignet sind; und
(b) Nachweisen des Vorliegens von Immunkomplexen in der Probe, wobei der Nachweis von Immunkomplexen das Vorliegen von *Chlamydia pneumoniae* in der Probe anzeigt.

18. DNA-Chip, der eine Anordnung von Polynukleotiden enthält, die mindestens eines der Polynukleotide nach den Ansprüchen 1 bis 3 umfasst.

19. Protein-Chip, der eine Anordnung von Polypeptiden enthält, die mindestens eines der Polypeptide nach Anspruch 9 oder 10 umfasst.

20. Immunogene Zusammensetzung, die das Polypeptid nach den Ansprüchen 9 bis 12 und einen pharmazeutisch annehmbaren Trägerstoff umfasst.

21. Immunogene DNA-Zusammensetzung, die den Expressionsvektor nach Anspruch 4 oder 5 umfasst.

22. DNA-Zusammensetzung nach Anspruch 21, wobei die DNA-Zusammensetzung die Expression eines neutralisierenden Epitops von *Chlamydia pneumoniae* steuert.

23. Pharmazeutische Zusammensetzung, die das Polypeptid nach den Ansprüchen 9 bis 12 oder einen Antikörper nach Anspruch 13 und einen pharmazeutisch annehmbaren Trägerstoff umfasst.

24. Zusammensetzung zur Behandlung oder verhütung einer Infektion mit *Chlamydia pneumoniae,* wobei die Zusammensetzung Folgendes umfasst:
a) ein Polynukleotid nach den Ansprüchen 1 bis 3;
b) ein Polypeptid nach den Ansprüchen 9 bis 12;
c) einen Antikörper nach Anspruch 13;
d) einen Vektor nach den Ansprüchen 4 und 5 oder
e) eine Wirtszelle nach den Ansprüchen 6 und 7.

25. Zusammensetzung nach Anspruch 24 als ein Impfstoff.

26. Screening-Assay, der Folgendes umfasst:
(a) Inkontaktbringen einer Testverbindung mit einem isolierten Polynukleotid nach den Ansprüchen 1 bis 3 oder mit einem isolierten Polypeptid nach den Ansprüchen 9 und 10 und
(b) Nachweisen, ob eine Bindung stattfindet.

27. Kit, der einen Behälter umfasst, der ein isoliertes Polynukleotid nach den Ansprüchen 1 bis 3 oder einen Primer oder eine Sonde, der bzw. die spezifisch mit einem Polynukleotid nach den Ansprüchen 1 bis 3 hybridisieren kann, ein isoliertes Polypeptid nach den Ansprüchen 9 und 10 oder einen Antikörper nach Anspruch 13 enthält.

## Revendications

1. Polynucléotide isolé ayant une séquence nucléotidique d'un cadre de lecture ouvert (ORF) d'un génome de *Chlamydia pneumoniae,* comprenant :
(a) la séquence nucléotidique ORF291 codant pour la séquence d'acides aminés SEQ ID N° : 291 ;
(b) une séquence nucléotidique présentant une identité d'au moins 80 % avec la séquence nucléotidique ORF291 ; ou
(c) une séquence complémentaire de celles-ci.

2. Polynucléotide selon la revendication 1, qui code pour les polypeptides suivants ou fragments de ceux-ci :
(a) un polypeptide de *Chlamydia pneumoniae* de l'enveloppe cellulaire, de préférence, de l'enveloppe cellulaire externe ;
(b) un polypeptide transmembranaire de *Chlamydia pneumoniae* ayant entre 1 et 3 domaines transmembranaires ;
(c) un polypeptide exposé à la surface de *Chlamydia pneumoniae ;*
(d) une lipoprotéine de *Chlamydia pneumoniae ;* ou
(e) un polypeptide de *Chlamydia pneumoniae* qui ne se trouve pas dans *Chlamydia trachomatis.*

3. Polynucléotide codant pour une protéine de fusion, comprenant le polynucléotide selon la revendication 1 ou 2, lié dans le cadre à un polynucléotide codant pour un polypeptide hétérologue.

4. Vecteur recombinant qui contient le polynucléotide selon l'une des revendications 1 à 3.

5. Vecteur recombinant qui contient le polynucléotide selon l'une des revendications 1 à 3, associé de manière opérationnelle à une séquence régulatrice qui contrôle l'expression du gène.

6. Cellule hôte génétiquement modifiée qui contient le polynucléotide selon l'une des revendications 1 à 3.

7. Cellule hôte génétiquement modifiée qui contient le polynucléotide selon l'une des revendications 1 à 3, associé de manière opérationnelle à une séquence régulatrice qui contrôle l'expression du gène dans la cellule hôte.

8. Méthode de production d'un polypeptide, comprenant
(a) la culture de la cellule hôte génétiquement modifiée selon l'une des revendications 6 ou 7, dans des conditions appropriées pour produire le polypeptide codé par le polynucléotide ; et
(b) la récupération du polypeptide de la culture.

9. Polypeptide codé par le polynucléotide selon l'une des revendications 1 à 3.

10. Polypeptide selon la revendication 9, qui comprend les polypeptides suivants ou leurs fragments :
(a) un polypeptide d'enveloppe cellulaire de *Chlamydia pneumoniae,* de préférence de l'enveloppe cellulaire externe ;
(b) un polypeptide transmembranaire de *Chlamydia pneumoniae* ayant entre 1 et 3 domaines transmembranaires ;
(c) un polypeptide exposé à la surface de *Chlamydia pneumoniae ;*
(d) une lipoprotéine de *Chlamydia pneumoniae ;* ou
(e) un polypeptide de *Chlamydia pneumoniae* qui ne se trouve pas dans *Chlamydia trachomatis.*

11. Protéine de fusion codée par le polynucléotide selon la revendication 9 ou 10.

12. Polypeptide selon l'une des revendications 9 à 11, qui immuno-réagit avec un sérum séropositif d'un individu infecté par *Chlamydia pneumoniae.*

13. Anticorps qui se lie de façon immunospécifique au polypeptide selon l'une des revendications 9 ou 10.

14. Méthode de détection et/ou d'identification de *Chlamydia pneumoniae* dans un échantillon biologique, comprenant :
(a) la mise en contact de l'échantillon avec une amorce polynucléotidique qui s'hybride spécifiquement avec le polynucléotide ORF291 ou une séquence complémentaire de celui-ci, en présence d'une enzyme polymérase et de nucléotides dans des conditions qui permettent l'extension de l'amorce ; et
(b) la détection de la présence de produits d'extension d'amorce dans l'échantillon, la détection des produits d'extension d'amorce indiquant la présence de *Chlamydia pneumoniae* dans l'échantillon.

15. Méthode de détection et/ou d'identification de *Chlamydia pneumoniae* dans un échantillon biologique, comprenant :
(a) la mise en contact de l'échantillon avec une sonde polynucléotidique qui s'hybride spécifiquement avec le polynucléotide ORF291 ou une séquence complémentaire de celui-ci, dans des conditions qui permettent l'hybridation de paires de bases complémentaires ; et
(b) la détection de la présence de complexes d'hybridation dans l'échantillon, la détection des complexes d'hybridation indiquant la présence de *Chlamydia pneumoniae* dans l'échantillon.

16. Méthode de détection et/ou d'identification de *Chlamydia pneumoniae* dans un échantillon biologique, comprenant :
(a) la mise en contact de l'échantillon avec l'anticorps selon la revendication 13, dans des conditions appropriées pour la formation de complexes immuns ; et
(b) la détection de la présence de complexes immuns dans l'échantillon, la détection des complexes immuns indiquant la présence de *Chlamydia pneumoniae* dans l'échantillon.

17. Méthode de détection et/ou d'identification d'anticorps contre *Chlamydia pneumoniae* dans un échantillon biologique, comprenant :
(a) la mise en contact de l'échantillon avec un polypeptide selon l'une des revendications 9 et 10, dans des conditions appropriées à la formation de complexes immuns ; et
(b) la détection de la présence de complexes immuns dans l'échantillon, la détection des complexes immuns indiquant la présence de *Chlamydia pneumoniae* dans l'échantillon.

18. Puce à ADN contenant un jeu de polynucléotides comprenant au moins l'un des polynucléotides selon l'une des revendications 1 à 3.

19. Puce à protéines contenant un jeu de polypeptides comprenant au moins l'un des polypeptides selon la revendication 9 ou 10.

20. Composition immunogène comprenant le polypeptide selon l'une des revendications 9 à 12 et un véhicule pharmaceutiquement acceptable.

21. Composition immunogène à ADN comprenant le vecteur d'expression selon la revendication 4 ou 5.

22. Composition à ADN selon la revendication 21, la composition à ADN dirigeant l'expression d'un épitope neutralisant de *Chlamydia pneumoniae.*

23. Composition pharmaceutique comprenant le polypeptide selon l'une des revendications 9 à 12, ou un anticorps selon la revendication 13 et un véhicule pharmaceutiquement acceptable.

24. Composition pour le traitement ou la prévention d'une infection à *Chlamydia pneumoniae,* ladite composition comprenant :
a) un polynucléotide selon l'une des revendications 1 à 3 ;
b) un polypeptide selon l'une des revendications 9 à 12 ;
c) un anticorps selon la revendication 13 ;
d) un vecteur selon l'une des revendications 4 et 5 ; ou
e) une cellule hôte selon l'une des revendications 6 et 7.

25. Composition selon la revendication 24 sous forme de vaccin.

26. test de criblage, comprenant
(a) la mise en contact d'un composé de test avec un polynucléotide isolé selon l'une des revendications 1 à 3, ou avec un polypeptide isolé selon l'une des revendications 9 et 10 ; et
(b) la détection de la formation de la liaison.

27. Kit comprenant un récipient contenant un polynucléotide isolé selon l'une des revendications 1 à 3 ou une amorce ou une sonde pouvant s'hybrider spécifiquement avec un polynucléotide selon l'une des revendications 1 à 3, un polypeptide isolé selon l'une des revendications 9 et 10, ou un anticorps selon la revendication 13.
